# EUROPEAN PATENT APPLICATION

(11) **EP 3 936 627 A1**
(43) Date of publication of application: **12.01.2022**
(21) Application number: 20184339.8
(22) Date of filing: 06.07.2020
(51) Int. Cl.: C12Q 1/689

(54) **NOVEL OLIGONUCLEOTIDES FOR DETECTING STAPHYLOCOCCUS**

(71) Applicant: Beiersdorf AG, 20253 Hamburg (DE)
(72) Inventor: HUEPEDEN, Jennifer, 20146 Hamburg (DE); FOELSTER, Heike, 22149 Hamburg (DE); REUTER, Jörn Hendrik, 24558 Henstedt-Ulzburg (DE); AHLE, Charlotte, 20259 Hamburg (DE)
(74) Representative: Uexküll & Stolberg

(57) **Abstract**

The present invention relates to the field of nucleic acid amplification. More particularly, the invention relates to oligonucleotide primers for amplifying the sequence or part of the sequence of a *Staphylococcus tuf* gene. In another aspect, the invention relates to a method for identifying one or more *Staphylococcus* species and/or strains which are present in a biological sample, such as a skin swab. In yet another aspect, the invention relates to the use of an oligonucleotide primer of the present invention for amplifying the sequence or part of the sequence of a *Staphylococcus tuf* gene. Kits which comprise an oligonucleotide primer of the present invention are also provided for carrying out the methods of the invention.

## Description

The present invention relates to the field of nucleic acid amplification. More particularly, the invention relates to oligonucleotide primers for amplifying the sequence or part of the sequence of a *Staphylococcus tuf* gene. In another aspect, the invention relates to a method for identifying one or more *Staphylococcus* species and/or strains which are present in a biological sample, such as a skin swab. In yet another aspect, the invention relates to the use of an oligonucleotide primer of the present invention for amplifying the sequence or part of the sequence of a *Staphylococcus tuf* gene. Kits which comprise an oligonucleotide primer of the present invention are also provided for carrying out the methods of the invention.

### BACKGROUND OF THE INVENTION

Coagulase-negative staphylococcal species (CoNS) constitute an important part of the human skin microbiome. In particular, facultative anaerobic species such as *Staphylococcus epidermidis* and *Staphylococcus capitis* can be found on the skin of almost every human being. Studies have highlighted the prevalence of CoNS, which colonize mostly moist and sebaceous areas of the skin. In this regard, the CoNS species *S. epidermidis, S. capitis* and *S. hominis* occupy virtually every human skin site [1, 2]. Other CoNS species such as *S. haemolyticus, S. warneri*, *S. cohnii, S. simulans, S. auricularis, S. lugdunensis, S. massiliensis* and *S. pettenkoferi* can be found in lower amounts, varying from person to person and skin site to skin site [3-7]. Some other CoNS species such as *S. equorum* are primary found in food products [8], but are also transient colonizers of human skin.

Culture-dependent approaches to identify all microbial skin inhabitants are often biased, which means that the results obtained from such studies do often not reflect the true distribution of the individual members of the skin microbiota [9]. The bias is introduced due to the chosen growth media, as well as the conditions of growth, such as O₂ and CO₂ concentrations, growth temperature and cultivation time. Fast-growing organisms usually have a growth advantage and may directly or indirectly inhibit the growth of slow-growing organisms, in particular microaerophilic or anaerobic species [10].

Accordingly, culture-independent studies employing next-generation sequencing (NGS) have been used more frequently in recent years. Using 16S rRNA amplicon NGS, it was shown that the genus *Staphylococcus* is the third most abundant genus on the skin [11]. More recently, shotgun NGS unraveled the diversity of staphylococcal species on the skin, and also resolved the strain-level distribution of species such as *S. epidermidis* [1]. Culture-independent studies have not only revealed the diversity and individuality of the skin microbiota, but also have identified microbial "dark matter". For example, shotgun NGS resulted in many sequences that could not be assigned to any known microbial species. For instance, the study reported in reference [1] identified several uncharacterized genomes (assembled from shotgun NGS data) that belonged to unknown species, possibly species of the genera *Corynebacterium*, *Cutibacterium*, and *Staphylococcus.* Thus, it can be expected that several species exist on the skin that cannot be easily cultivated by standard conditions.

The detection and quantification of bacterial species, and in particular *Staphylococcus* species, in the skin microbiome of an individual is of utmost importance for understanding the mechanisms which contribute to the development of skin diseases or conditions like acne. It is assumed that in healthy skin, the different *Staphylococcus* species are well balanced with respect to each other and that disturbing this balance is causative for or at least contributes to the development of diseases. Accordingly, it is important to detect the different *Staphylococcus* species that occur on the skin of an individual and to determine their relative abundances to be able to provide methods for treating, ameliorating or preventing skin conditions which are tailored to the respective individual. Stated differently, the overall objective is a step towards personalized skin care.

Amplicon NGS approaches available to date for determining *Staphylococcus* species have made an essential contribution to reaching this objective. However, further improvements are required on the level of the means and methods that are used for detection. For example, it has been found that the primers used in commonly available amplicon NGS methods are able to detect only some species reliably while other species are not detected at all. Amplicon NGS techniques used for determining the *Staphylococcus* species in a given sample therefore provide an incomplete and hence incorrect picture of the microbiome that is analyzed. Therefore, methods are needed which allow for a more diverse detection of *Staphylococcus* species in a sample.

### DESCRIPTION OF THE INVENTION

It has been found herein that by selecting appropriate primers, it is possible to obtain a more complete picture of the different *Staphylococcus* species and their relative abundances in a biological sample. These primers target the *tuf* gene, a gene that is present in all *Staphylococcus* species and encodes the elongation factor Tu. The primers described herein have been found to amplify part of the *tuf* gene sequence of essentially all *Staphylococcus* species that commonly occur on human skin. Consequently, it is possible to employ these primers in a universal approach that aims at the identification of all *Staphylococcus* species that are commonly present in a sample to be analyzed.

By using the primers of the invention, it was surprisingly found that numerous skin samples comprise the species *S. saccharolyticus* which appears to be a common member of the skin microbiome. This species has rarely been described to date; it was found on a few skin samples [13, 14], and like several other CoNS, the species has been described in case studies in association with a few infections in humans, such as bacteremia [15], soft tissue, bone and joint infections and implant-associated infections [12]. In the present studies using the novel primers, *S. saccharolyticus* was identified as the third most abundant species on the skin of the back. Although described as a slow-growing anaerobic species, a re-evaluation of its growth behaviour showed that this species can grow under oxic conditions in the presence of 5% CO₂. It can therefore be assumed that *S. saccharolyticus* was largely overlooked in previous studies due to the requirement for fastidious growth conditions.

This demonstrates that prior art methods have not been able so far to correctly reflect the level of diversity within the group of *Staphylococcus* bacteria that populate the human skin. As a result, these primers are disadvantageous when used for determining the composition of the skin microbiome. The primers of the present invention enable a more specific analysis in that they detect species that so far escaped analysis.

Thus, in a first aspect, the invention relates to an oligonucleotide primer comprising:
a) the nucleotide sequence of SEQ ID NO:1 or SEQ ID NO:2; or
b) a sequence which is at least 85% identical to the nucleotide sequence of SEQ ID NO:1 or SEQ ID NO:2; or
c) the complement of (a) or (b).

In one preferred embodiment of the invention, the primer comprises the nucleotide sequence of SEQ ID NO:1 or its complement. In another preferred embodiment, the primer comprises the nucleotide sequence of SEQ ID NO:2 or its complement. In addition, as will be understood by those skilled in the art, a limited degree of sequence variation relative to the nucleotide sequence of SEQ ID NO:1 or SEQ ID NO:2 or their complements may occur without affecting the functionality of the oligonucleotide primers. For example, sequence variants may be used having one or more (e.g. 1, 2, 3) substitutions, insertions or deletions of nucleotides relative to the sequence of SEQ ID NO:1 or SEQ ID NO:2 or their complements. The variant will have at least 85% sequence identity to the sequence of SEQ ID NO:1 or SEQ ID NO:2 or their complements, preferably at least 86%, 87%, 88%, 89%, 90%, 91%, 92%, 93%, 94%, 95%, 96%, 97%, 98%, or 99% sequence identity. In a particularly preferred aspect of the invention, the oligonucleotide probe consists of the sequence of SEQ ID NO:1 or SEQ ID NO:2.

In a particularly preferred embodiment, the invention relates to an oligonucleotide primer that comprises or consists of
a) the nucleotide sequence of SEQ ID NO:1 or SEQ ID NO:2; or
b) a sequence which differs from SEQ ID NO:1 or SEQ ID NO:2 by no more than 2 nucleotides , and more preferably no more than 1 nucleotide; or
c) the complement of (a) or (b).

As used herein, the term oligonucleotide denotes a molecule consisting of at least 5 nucleotides, and preferably at least 10, 15, 20, 25, 30 or 35 nucleotides. The length of the molecule will be up to 35 nucleotides, preferably up to 40, 45, 50, 55, 60, 65, 70, 75 or 80 nucleotides. In other words, an oligonucleotide as defined herein will have a length of 10-70, more preferably 10-50, 15-50, 20-50 or 25-50 nucleotides.

The oligonucleotide primer of the present invention may include one or more sequences that are not complementary to the *Staphylococcus* target sequence, such as sequencing adaptors. In a preferred embodiment, the primer of the invention comprises adapter sequences for NGS. Suitable NGS adapter sequences are known in the art and comprise, e.g. the adapters used in the Illumina NGS sequencing kits.

Primer sequences with NGS adapter sequences are exemplary set forth in SEQ ID NO:3 and SEQ ID NO:4. Thus, the invention also relates to an oligonucleotide primer comprising:
a) the nucleotide sequence of SEQ ID NO:3 or SEQ ID NO:4; or
b) a sequence which is at least 85% identical to the nucleotide sequence of SEQ ID NO:3 or SEQ ID NO:4; or
c) the complement of (a) or (b).

In a preferred embodiment, the invention relates to an oligonucleotide primer comprising or consisting of:
a) the nucleotide sequence of SEQ ID NO:3 or SEQ ID NO:4; or
b) a sequence which differs from SEQ ID NO:3 or SEQ ID NO:4 by no more than 2 nucleotides , and more preferably no more than 1 nucleotide; or
c) the complement of (a) or (b).

In a particularly preferred embodiment of the invention, the primer of the invention consists of a sequence of any of SEQ ID NOs:1-4. The primers depicted in SEQ ID NOs:1 and 3 are forward primers which can be combined with corresponding reverse primers so as to form a primer pair that can be used for the amplification of the target sequence. The primers depicted in SEQ ID NOs:2 and 4 are reverse primers. Preferably, the primer of SEQ ID NO:1 is used in combination with the primer of SEQ ID NO:2, and the primer of SEQ ID NO:3 is used in combination with the primer of SEQ ID NO:4.

In a second aspect, the invention relates to a method for amplifying the sequence or part of the sequence of a *Staphylococcus tuf* gene, said method comprising:
a) obtaining nucleic acid from bacteria of the genus *Staphylococcus,* preferably genomic DNA;
b) amplifying the nucleic acid with at least one oligonucleotide primer as defined hereinabove; and
c) optionally, sequencing the amplified nucleic acid.

In a first step of the above method, nucleic acid is obtained from bacteria of the genus *Staphylococcus.* The nucleic acid can be DNA, RNA or a mixture of DNA and RNA. Preferably, the nucleic acid which is subjected to the method of the invention will comprise DNA derived from bacteria of the genus *Staphylococcus,* more preferably genomic DNA. The nucleic acid used for amplification preferably contains nucleic acids, preferably genomic DNA molecules, from more than one *Staphylococcus* species or strain, i.e. from two, three, four, five or more *Staphylococcus* species or strains. This means that the nucleic acid used in step (a) of the method of the second aspect of the invention can be a mixture of nucleic acids derived from different *Staphylococcus* species or strains.

For example, the nucleic acid may be genomic DNA isolated from a group of bacteria that are present colonizes a tissue, a tissue surface, e.g. the skin surface, or a body fluid of a subject, preferably a human subject. This means that the nucleic acid can be a mixture of nucleic acids of the bacteria that form the microbiome of a specific tissue, tissue surface, or body fluid. As used herein, the term microbiome refers to the entirety of bacterial species that colonize a particular area of the skin, e.g. the facial skin. In a particular preferred embodiment, the nucleic acid used in step (a) of the method of the second aspect of the invention is genomic DNA of the microbiome of the human skin, more preferably the human facial skin or the skin of the back or armpit. In other embodiments, the nucleic acid is genomic DNA of the microbiome of a body fluid. Such body fluid may include whole blood, blood serum, blood plasma, urine, sputum, bronchial lavage, liquor, and the like.

Samples of the microbiome of a tissue or body fluid can be obtained by commonly known methods, e.g. biopsy, venipuncture and nasal or skin swabs. In a particularly preferred embodiment, the nucleic acid sample is genomic DNA isolated from the microbiome of the human skin, such as the facial skin or the skin of the back or armpit. The microbiome sample preferably is a nasal or skin swab.

After the microbiome sample has been obtained from the individual, e.g. a skin swab, the nucleic acid of the bacteria that are present in the sample can be obtained by using commonly available kits, e.g. kits for the purification of DNA or RNA (available e.g. from Qiagen, Hilden, Germany). These kits normally provide for the lysis of the bacterial cells in the sample, selective binding of the genomic DNA to a matrix, purification of the bound DNA by removal of proteins and other cell components, and elution of the purified genomic DNA. Methods of isolating and purifying genomic DNA from bacteria are well known to a person of skill.

In a subsequent step of the method of the second aspect of the invention, the nucleic acid obtained in step (a) is amplified by use of at least one oligonucleotide primer as defined hereinabove, i.e. an oligonucleotide primer comprising:
a) the nucleotide sequence of any of SEQ ID NOs:1-4;
b) a sequence which is at least 85% identical to the nucleotide sequence of any of SEQ ID NOs:1-4 or a sequence which differs from of any of SEQ ID NOs:1-4 by no more than 2 nucleotides, and more preferably no more than 1 nucleotide; or
c) the complement of (a) or (b).

In a preferred embodiment, the nucleic acid is amplified in step (b) by using both (i) a primer comprising the nucleotide sequence of SEQ ID NO:1 (or the complement thereof) or a sequence which is at least 85% or at least 90% identical to any of those, and (ii) a primer comprising the nucleotide sequence of SEQ ID NO:2 (or the complement thereof) or a sequence which is at least 85% or at least 90% identical to any of those. Accordingly, it is preferred that the nucleic acid is amplified in step (b) with
(i) a first primer comprising:
   a) the nucleotide sequence of SEQ ID NO:1; or
   b) a sequence which is at least 85%, more preferably at least 90%, identical to the nucleotide sequence of SEQ ID NO:1 or which differs from SEQ ID NO:1 by no more than 1 or 2 nucleotides; or
   c) the complement of (a) or (b); and
(ii) a second primer comprising:
   a) the nucleotide sequence of SEQ ID NO:2; or
   b) a sequence which is at least 85%, more preferably at least 90%, identical to the nucleotide sequence of SEQ ID NO:2 or which differs from SEQ ID NO:2 by no more than 1 or 2 nucleotides; or
   c) the complement of (a) or (b).

Where the nucleic acid in the nucleic acid sample is DNA, the amplification can be achieved by a polymerase chain reaction (PCR). A PCR is an enzymatic reaction for increasing the amount or concentration of a DNA sequence which is catalyzed by a thermostable DNA-dependent DNA polymerase. The primers are complementary to one strand of the double-stranded target sequence. For amplification, the double-stranded target sequence is denatured so as to allow annealing of the primers. Following annealing, the primers are extended by means of the DNA polymerase to form a new pair of complementary strands. The steps of denaturation, primer annealing, and polymerase extension are repeated several times, e.g. 15-35 times, to obtain a high amount or concentration of the target DNA sequence. PCR amplification methods are well-known to the skilled person, and PCR kits are purchasable from many different manufacturers. The oligonucleotide primers defined above can be used for amplification of a part of the *Staphylococcus tuf* gene.

Alternatively, the oligonucleotide primers of the present invention may also be used in an RT-PCR reaction. Where the nucleic acid extracted from the biological sample is RNA (e.g. total RNA or mRNA), the first step of the amplification is a reverse transcription reaction (RT). In this reaction, the RNA is transcribed into cDNA by means of a reverse transcriptase enzyme. Reverse transcription can be achieved by use of a sequence specific primer or an oligo-d(T) primer which is complementary to the poly (A) tail of the RNA. The cDNA generated by the reverse transcriptase is then used as a template in a subsequent PCR reaction. An amplification reaction comprising a reverse transcription reaction and a subsequent PCR amplification reaction is referred to as "RT-PCR" herein. According to the present invention, the RT-PCR can be performed as a one-step or two-step RT-PCR. The primers defined above can be used for specifically amplifying the *Staphylococcus tuf* gene when starting from a RNA preparation that was derived from a microbiome that is colonized by *Staphylococcus* species and/or strains.

In an optional step of the method of the second aspect of the invention, the amplified nucleic acid obtained from step (b) is subjected to sequencing which means that the nucleotide sequence of the amplicons derived from step (b) is determined. In principle, any method known in the art for sequencing nucleic acids, in particular DNA, can be used including Maxam-Gilbert sequencing, Sanger sequencing, Shotgun sequencing, and next-generation sequencing (NGS), pyrosequencing, nanopore sequencing and the like. In a preferred embodiment, the sequencing step is performed by NGS, for example, by the sequencing-by-synthesis (SBS) technology of Illumina. Sequencing kits for NGS are available from many different manufacturers.

In a third aspect of the invention, the invention further relates to a method for identifying one or more *Staphylococcus* species and/or strains which are present in a biological sample, comprising
a) obtaining a nucleic acid from bacteria of the genus *Staphylococcus,* preferably genomic DNA;
b) amplifying the nucleic acid with at least one oligonucleotide primer as described hereinabove;
c) sequencing the amplified nucleic acid;
d) comparing the sequences obtained in step (c) with reference sequences from different *Staphylococcus* species and/or strains; and
e) assigning the sequences obtained in step (c) to a *Staphylococcus* reference sequence, thereby identifying the species and/or strains.

Accordingly, the method of the third aspect of the invention comprises all steps of the method of the second aspect of the invention (with the sequencing step being mandatory) and, in addition, steps in which the sequences are compared to reference sequences, i.e. known sequences of the *tuf* gene of a defined *Staphylococcus* species and/or strain.

Steps (a)-(c) of the method of the third aspect of the invention are carried out as described in relation to steps (a)-(c) of the method of the second aspect of the invention.

In the subsequent step (d) of the above method of the third aspect of the invention, the nucleic acid sequences determined in step (c) are compared to reference sequences from a plurality of different *Staphylococcus* species and/or strains. Preferably, the sequences determined in step (c) can be compared to a database that harbours the nucleotide sequences of a plurality of *tuf* genes derived from different *Staphylococcus* species and/or strains. Preferably, the database will contain at least 10, least 20, least 30, least 40, least 50, least 60, least 70, least 80 or more different complete or partial *tuf* gene sequences derived from different *Staphylococcus* species and/or strains. A suitable database is provided in the attached sequence listing that comprises the nucleotide sequences of 96 *tuf* genes. Accordingly, in a preferred embodiment, the sequences determined in step (c) of the above method are compared with two or more of the reference sequences set forth in SEQ ID NOs:5-100, more preferably with at least 5, at least 10, at least 15, at least 20, at least 25, at least 30, at least 35, at least 40, at least 45, at least 50, least 55, at least 60, at least 65, at least 70, at least 75, least 80, at least 85, at least 90, at least 95 or more of these sequences. It is particularly preferred that the sequences determined in step (c) of the method of the third aspect of the invention are compared with a database which harbours all of the nucleotide sequences set forth in SEQ ID NOs:5-100.

Of course, the sequences determined in step (c) can also be compared to a freely accessible database that comprises all different types of genomic DNA sequences from different bacterial organisms, such as the GenBank database.

The comparison will allow assigning a sequence determined in step (c) of the method to a specific *Staphylococcus* reference sequence derived from a defined *Staphylococcus* species or strain, thereby identifying the species and/or strains. For example, a nucleotide sequence determined in step (c) of the claimed method can be assigned to a specific *Staphylococcus* reference sequence if the sequence identity on the nucleotide level is at least 98%, preferably at least 99%. In a particularly preferred embodiment, the nucleotide sequence determined in step (c) of the above method can be assigned to a specific *Staphylococcus* reference sequence if the sequence identity on the nucleotide level is 100%.

Preferably, the sequence identity is determined over a length of at least 50 nucleotides, more preferably at least 100 nucleotides, at least 150 nucleotides, at least 200 nucleotides, at least 250 nucleotides, at least 300 nucleotides, at least 350 nucleotides, at least 400 nucleotides, or at least 450 nucleotides of the *tuf* gene.

In one preferred embodiment, the nucleotide sequence determined in step (c) of the claimed method can be assigned to a specific *Staphylococcus* reference sequence if the sequence identity on the nucleotide level is at least 98% over a length of at least 200 nucleotides, more preferably at least 300 nucleotides, and even more preferably at least 400 nucleotides. For example, the sequence identity of at least 98% is measured over a length of between 200-400 nucleotides, preferably between 200-300 nucleotides.

In another preferred embodiment, the nucleotide sequence determined in step (c) of the claimed method can be assigned to a specific *Staphylococcus* reference sequence if the sequence identity on the nucleotide level is at least 99% over a length of at least 200 nucleotides, more preferably at least 300 nucleotides, and even more preferably at least 400 nucleotides. For example, the sequence identity of at least 99% is measured over a length of between 200-400 nucleotides, preferably between 200-300 nucleotides.

In yet another preferred embodiment, the nucleotide sequence determined in step (c) of the claimed method can be assigned to a specific *Staphylococcus* reference sequence if the sequence identity on the nucleotide level is at least 100% over a length of at least 200 nucleotides, more preferably at least 300 nucleotides, and even more preferably at least 400 nucleotides. For example, the sequence identity of at least 100% is measured over a length of between 200-400 nucleotides, preferably between 200-300 nucleotides.

Sequence identity between two or more nucleotide sequences can be determined by using mathematical algorithms. Algorithm used sequences comparison are well know in the art and include the algorithm of Karlin and Altschul, 1990, Proc. Natl. Acad. Sci. USA 87:2264-2268, and the modified algorithm of Karlin and Altschul, 1993, Proc. Natl. Acad. Sci. USA 90:5873-5877. These algorithms are incorporated into the NBLAST and XBLAST programs of Altschul et al., 1990, J. Mol. Biol. 215:403-410. BLAST nucleotide searches can be performed with the NBLAST program, score=100, wordlength=12, to obtain nucleotide sequences homologous to a nucleic acid of interest. Another example of a mathematical algorithm that can be used for the comparison of nucleotide sequences is the algorithm of Myers and Miller, CABIOS (1989). This algorithm is used in the ALIGN program (version 2.0) which is part of the GCG sequence alignment software package. Additional algorithms for sequence analysis include FASTA (Pearson and Lipman, 1988, Proc. Natl. Acad. Sci. USA 85:2444-8).

In order to determine the sequence identity between two nucleotide sequences, these sequences are usually aligned for optimal comparison. For example, gaps can be introduced in the sequence of a first nucleotide sequence for optimal alignment with a second nucleotide sequence. The nucleotides at corresponding nucleotide positions are then compared. If identical nucleotides occur in corresponding positions in the first and second nucleotide sequence, the sequences are identical at that position.

By assigning all of the sequences identified in step (c) of the above method of the third aspect of the invention to a single *Staphylococcus* reference sequence of a defined species or strain, it is possible to determine all the different *Staphylococcus* species and/or strains that occur within the microbiome from which the ample was derived. In this way, it is possible to identify essentially all of the distinct *Staphylococcus* species and/or strains that occur, e.g. on a defined skin area of an individual, such as the facial skin.

Apart from a merely qualitative identification in step (e), the method can also b adapted to provide a quantitative identification. As used herein, a "qualitative" identification means that the different nucleotide sequences determined in step (c) of the method are assigned to a defined *Staphylococcus* species and/or strain such that all species and/or strains present in the original sample are determined. In contrast, a "quantitative" identification means that not only the occurrence of a species and/or strain I determined, but also the relative or absolute abundance of the respective species and/or strain.

For example, it will be advantageous under certain conditions to determine the relative abundance of a *Staphylococcus* species on the skin. For example, it is known that a high concentration of the species *S. hominis* on the skin of the armpit is regularly associated with an unpleasant sweat odour, whereas *S. epidermidis* produces significantly less odour. Accordingly, knowledge of the relative abundances of *S. hominis* and *S. epidermidis* on this particular skin area of an individual can assist in the selection of a deodorant that is most suitable for the individual. For example, if the above method of the third aspect of the invention reveals that the concentration of *S. hominis* is high, a stronger deodorant can be selected for this individual. In this way, the method can contribute to a personalized skin care.

In a fourth aspect, the invention also relates to the use of an oligonucleotide primer as defined above for amplifying the sequence or part of the sequence of a *Staphylococcus tuf* gene. Such use may include, as indicated elsewhere herein, the amplification of nucleic acid obtained from a mixture of *Staphylococcus* species and/or strains in order to identify and distinguish those species and/or strains from each other. More particularly, the invention also relates to the use of an oligonucleotide primer as defined above for determining the composition of the *Staphylococcus* population that occurs within a given microbiome of an individual, i.e. the microbiome of the facial skin. In this way, it is possible to tailor skin care compositions in accordance with the presence and abundances of different *Staphylococcus* species and/or strains in a defined skin area of the individual. As explained elsewhere herein, the determination can be a qualitative and/or quantitative determination.

In a fifth aspect, the invention relates to a method for identifying the presence of *Staphylococcus saccharolyticus* in a biological sample, said method comprising
a) obtaining a nucleic acid from a biological sample that is supposed to comprise bacteria of the genus *Staphylococcus,* preferably genomic DNA;
b) amplifying the nucleic acid with at least one oligonucleotide primer as described hereinabove;
c) sequencing the amplified nucleic acid;
d) comparing the sequences obtained in step (c) with reference sequences from different *Staphylococcus* species and/or strains; and
e) assigning the sequences obtained in step (c) to a *Staphylococcus saccharolyticus* reference sequence, thereby identifying the presence of *Staphylococcus saccharolyticus* in the biological sample.

As explained elsewhere herein, the biological sample can be a tissue sample, such as a tissue biopsy or swab, or a body fluid sample, such as whole blood, blood serum, blood plasma, urine, sputum, bronchial lavage, liquor, and the like. Preferably, the biological sample is a tissue swab, such as a swab of the facial skin or the skin of the back or armpit.

In a sixth aspect, the invention relates to a kit for amplifying the sequence or part of the sequence of a *Staphylococcus tuf* gene, said kit comprising
a) at least one oligonucleotide primer as described hereinabove; and
b) means for performing a nucleic acid amplification and/or sequencing reaction.

The invention also pertains to a kit for carrying out any of the methods referred to hereinabove. The kit comprises at least one of the oligonucleotide primers of the invention, e.g. an oligonucleotide primer comprising or consisting of the nucleotide sequence of SEQ ID NO:1 or an oligonucleotide primer comprising or consisting of the nucleotide sequence of SEQ ID NO:2. Preferably, the kit includes a first oligonucleotide primer comprising: a) the nucleotide sequence of SEQ ID NO:1; or b) a sequence which is at least 85%, preferably at least 85%, preferably at least 90%, identical to the nucleotide sequence of SEQ ID NO:1; or c) the complement of (a) or (b); and a second primer comprising a) the nucleotide sequence of SEQ ID NO:2; or b) a sequence which is at least 85%, preferably at least 90%, identical to the nucleotide sequence of SEQ ID NO:2; or c) the complement of (a) or (b).

In a most preferred embodiment, the kit includes a first oligonucleotide primer comprising the nucleotide sequence of SEQ ID NO:1 or a complement thereof and a second oligonucleotide primer comprising the nucleotide sequence of SEQ ID NO:2 or a complement thereof. The kit also includes means for performing a nucleic acid amplification and/or a sequencing reaction. Preferably, the kit will comprise buffers and reagents that are suitable for amplifying a bacterial nucleic acid, such as DNA. The kit may include, for example, suitable buffers or enzymes like one or more polymerases. The kit may also include suitable sequencing adapters for NGS sequencing.

### BRIEF DESCRIPTION OF THE FIGURES

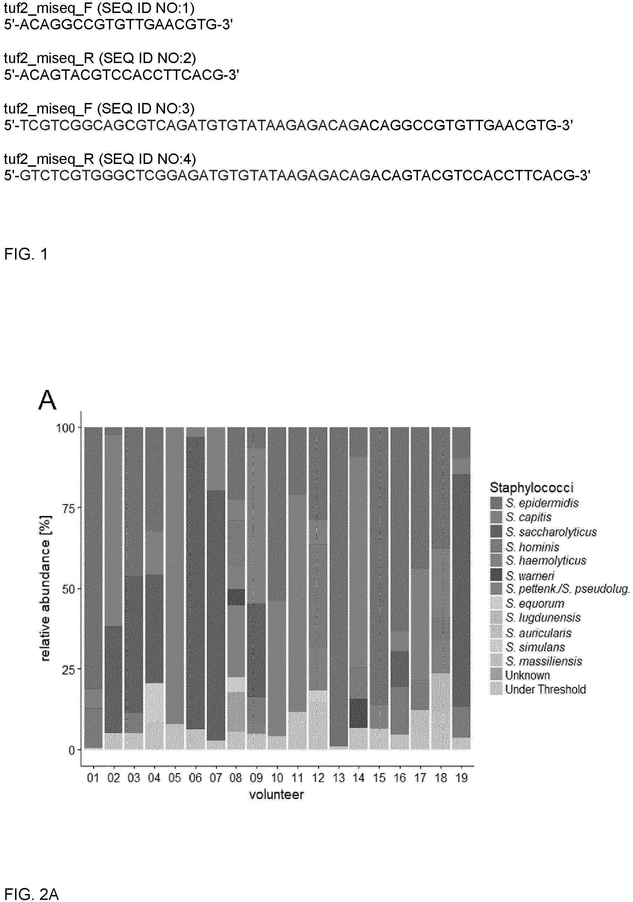

Figure 1 shows the sequence of the PCR primer used for amplifying the *tuf* gene. The *tuf-*specific sequence parts of these primers are depicted in SEQ ID NO:1 und SEQ ID NO:2. The primers that include the MiSeq adapter sequences are depicted in SEQ ID NO:3 und SEQ ID NO:4.

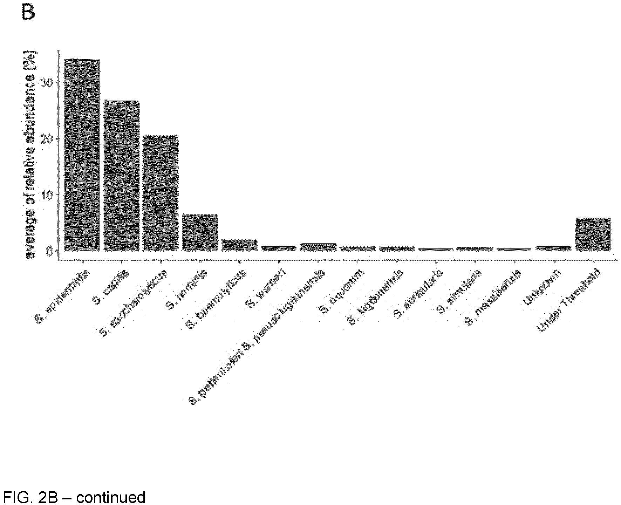

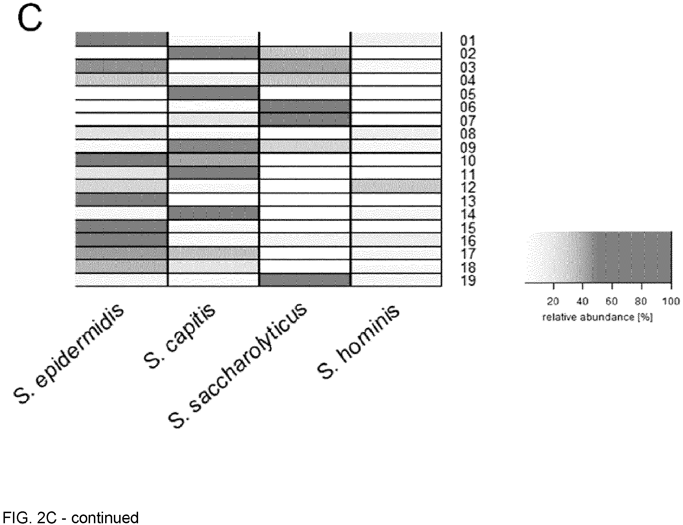

Figure 2 shows the results of the diversity analysis of staphylococcal species in back skin samples based on amplicon NGS data. Fig. 2A shows the relative abundancies of species for each volunteer. Fig. 2B shows the average of relative abundancies of 12 staphylococcal species. Fig. 2C shows the relative abundancies of the four most prominent species.

### EXAMPLES

The present invention is further illustrated by the following examples, which in no way should be construed as limiting. The entire contents of all of the references (including literature references, issued patents, published patent applications, and co pending patent applications) cited throughout this application are hereby expressly incorporated by reference.

### Example 1: Skin sample preparation and processing

Skin swab samples from 19 volunteers (female, n=11; male, n =8) with an age range of 22-43 years were taken from the upper back. None of the volunteers had a history of skin disease; none had undergone treatment with topical medicine or antibiotics during the last six months. Written informed consent was obtained from all volunteers and the study was approved by International Medical & Dental Ethics Commission GmbH (IMDEC).

An area of 25 cm² on the upper back was sampled with a cotton swab pre-moistened in aqueous sampling buffer containing disodium phosphate (12.49 g/L, Merck), potassium dihydrogen phosphate (0.63 g/L, Merck) and Triton X-100 (1 g/L, Sigma). After sampling, the swab was transferred into a sterile tube containing 2 mL of sampling buffer; the swap was viscously shacked in the sampling buffer and then removed. Skin swab material was stored at -20 °C until further processing.

DNA was extracted from the 2 mL sample by use of the DNeasy PowerSoil Kit (QIAGEN) following the manufacturer's protocol, with an additional cell lysis step with lysostaphin (0.05 mg/mL, Sigma) and lysozyme (9.5 mg/mL, Sigma) prior to extraction. DNA concentrations were measured by using Qubit with the Qubit dsDNA HS Assay (ThermoFisher Scientific). The DNA obtained from the extraction process was amplified using *tuf*-specific primers that contained MiSeq adapter sequences:
tuf2_miseq_F red (SEQ ID NO:1)
   5'-ACAGGCCGTGTTGAACGTG-3'
tuf2_miseq_R red (SEQ ID NO:2)
   5'-ACAGTACGTCCACCTTCACG-3'
tuf2_miseq_F (SEQ ID NO:3)
   5'-TCGTCGGCAGCGTCAGATGTGTATAAGAGACAGACAGGCCGTGTTGAACGTG-3'
tuf2_miseq_R (SEQ ID NO:4)
   5'-GTCTCGTGGGCTCGGAGATGTGTATAAGAGACAGACAGTACGTCCACCTTCACG-3'

The primers used for the PCR reaction are shown in SEQ ID NO:3 und SEQ ID NO:4. The *tuf*-specific sequence parts of these primers are depicted in SEQ ID NO:1 und SEQ ID NO:2. The primers are also shown in Fig. 1. PCR reaction mixtures were made in a total volume of 25 µl and comprised 5 µl of DNA sample, 2.5 µl AccuPrime PCR Buffer II (Invitrogen), 1.5 µl of each primer (10 µM) (DNA Technology), 0.15 µl AccuPrime Taq DNA Polymerase High Fidelity (Invitrogen), and 14.35 µl of PCR grade water. PCR was performed using the following cycle conditions: an initial denaturation at 94°C for 2 min, followed by 35 cycles of denaturation at 94°C for 20 sec, annealing at 55°C for 30 sec, elongation at 68°C for 1 min, and a final elongation step at 72°C for 5 min. The PCR products were verified on an agarose gel and purified using the Qiagen Generead^{™} Size Selection kit. The concentration of the purified PCR product was measured with a NanoDrop 2000 spectrophotometer (Thermo Scientific).

### Example 2: Next generation sequencing

The PCR products obtained in Example 1 were used to attach indices and Illumina sequencing adapters using the Nextera XT Index kit (Illumina, San Diego). Index PCR was performed using 5 µl of template PCR product, 2.5 µl of each index primer, 12.5 µl of 2x KAPA HiFi HotStart ReadyMix and 2.5 µl PCR grade water. Thermal cycling scheme was as follows: 95°C for 3 min, 8 cycles of 30s at 95°C, 30s at 55°C and 30s at 72°C and a final extension at 72°C for 5 min. Quantification of the products was performed using the Quant-iT dsDNA HS assay kit and a Qubit fluorometer following the manufacturer's instructions. MagSi-NGS^{PREP} Plus Magnetic beads (Steinbrenner Laborsysteme GmbH, Wiesenbach, Germany) were used for purification of the indexed products as recommended by the manufacturer and normalization was performed using the Janus Automated Workstation from Perkin Elmer (Perkin Elmer, Waltham Massachusetts, USA). Sequencing was conducted using Illumina MiSeq platform using dual indexing and MiSeq reagent kit v3 (600 cycles) as recommended by the manufacturer.

FASTQ sequences obtained after demultiplexing the reads and trimming the primers were imported into QIIME2 (v. 2019.7) [16]. Sequences with average quality score lower than 20 or containing unresolved nucleotides were removed from the dataset with the split_libraries_fastq.py script from QIIME. The paired-end reads were denoised and chimeras removed with DADA2 via QIIME2 and a feature table was generated [17]. These features were then clustered with VSEARCH at a threshold of 99% identity against an in-house generated tuf allele database that covered all tuf alleles from all staphylococcal genomes available in GenBank (as of December 2019). Data were normalized and figures were prepared in R with the packages ggplot2 [18] and gplots [19].

By the above approach, a total of twelve different staphylococcal species were identified on the back skin of the test persons (Figure 2A). Most samples contained two or more staphylococcal species. The back skin of only two volunteers harboured a single species only, *S. capitis* and *S. epidermidis,* respectively. The four most abundant staphylococcal species identified were *S. epidermidis* (average abundancy 34.0 %) and *S. capitis* (26.6 %), followed by *S. saccharolyticus* (20.5 %) and *S. hominis* (6.5 %). This is shown in Fig. 2B. In eight out of 19 samples (42 %) *S. saccharolyticus* could be identified. If present in the back skin sample, *S. saccharolyticus* was a dominant species; from 10.9 % up to 90.4 % of the total reads were originating from *S. saccharolyticus* in such samples (Fig. 2C).

### LITERATURE

1. Oh, J., et al., Biogeography and individuality shape function in the human skin metagenome. Nature, 2014. 514(7520): p. 59-64.
2. Kloos, W.E. and M.S. Musselwhite, Distribution and persistence of Staphylococcus and Micrococcus species and other aerobic bacteria on human skin. Appl Microbiol, 1975. 30(3): p. 381-5.
3. Schleiferi, K.H.K., W. E., Isolation and Characterization of Staphylococci from Human Skin I. Amended Descriptions of Staphylococcus epidermidis and Staphylococcus saprophyticus and Descriptions of Three New Species: Staphylococcus cohnii, Staphylococcus haemolyticus, and Staphylococcus xylosus. INT J SYST EVOL MICR, 1975. 25(1): p. 50-61.
4. Kloos, W.E.S., K. H., Staphylococcus auricularis sp. nov. : an Inhabitant of the Human External Ear. Int J Syst Bacteriol, 1983. 33(1): p. 9-14.
5. Bieber, L. and G. Kahlmeter, Staphylococcus lugdunensis in several niches of the normal skin flora. Clin Microbiol Infect, 2010. 16(4): p. 385-8.
6. Zong, Z., The newly-recognized species Staphylococcus massiliensis is likely to be part of the human skin microflora. Antonie Van Leeuwenhoek, 2012. 101(2): p. 449-51.
7. Mansson, E., et al., Genomic relatedness of Staphylococcus pettenkoferi isolates of different origins. J Med Microbiol, 2017. 66(5): p. 601-608.
8. Meugnier, H., et al., Identification and ribotyping of Staphylococcus xylosus and Staphylococcus equorum strains isolated from goat milk and cheese. Int J Food Microbiol, 1996. 31(1-3): p. 325-31.
9. Dickson, R.P., et al., Analysis of culture-dependent versus culture-independent techniques for identification of bacteria in clinically obtained bronchoalveolar lavage fluid. J Clin Microbiol, 2014. 52(10): p. 3605-13.
10. Kong, H.H. and J.A. Segre, Skin microbiome: looking back to move forward. J Invest Dermatol, 2012. 132(3 Pt 2): p. 933-9.
11. Grice, E.A., et al., Topographical and temporal diversity of the human skin microbiome. Science, 2009. 324(5931): p. 1190-2.
12. Bruggemann, H., et al., Staphylococcus saccharolyticus Isolated From Blood Cultures and Prosthetic Joint Infections Exhibits Excessive Genome Decay. Front Microbiol, 2019. 10: p. 478.
13. Evans, C.A., K.L. Mattern, and S.L. Hallam, Isolation and identification of Peptococcus saccharolyticus from human skin. J Clin Microbiol, 1978. 7(3): p. 261-4.
14. Evans, C.A. and K.L. Mattern, Individual differences in the bacterial flora of the skin of the forehead: Peptococcus saccharolyticus. J Invest Dermatol, 1978. 71(2): p. 152-3.
15. Steinbrueckner, B., et al., Facing a mysterious hospital outbreak of bacteraemia due to Staphylococcus saccharolyticus. J Hosp Infect, 2001. 49(4): p. 305-7.
16. Bolyen, E., et al., Reproducible, interactive, scalable and extensible microbiome data science using QIIME 2. Nat Biotechnol, 2019. 37(8): p. 852-857.
17. Callahan, B.J., et al., DADA2: High-resolution sample inference from Illumina amplicon data. Nat Methods, 2016. 13(7): p. 581-3.
18. Wickham, H., ggplot2: Elegant Graphics for Data Analysis. Springer-Verlag New York, 2009.
19. Warnes, G.R.B., B.; Bonebakker, L.; Gentleman, R.; Huber, W.; Liaw, A.; Lumley, T.; Maechler, M.; Magnusson, A.; Moeller, S.; Schwartz, M.; Venables, B., gplots: Various R Programming Tools for Plotting Data. 2020.
20. Oh, J., et al., Temporal Stability of the Human Skin Microbiome. Cell, 2016. 165(4): p. 854-66.
21. Strube, M.L., et al., A detailed investigation of the porcine skin and nose microbiome using universal and Staphylococcus specific primers. Sci Rep, 2018. 8(1): p. 12751.
22. Martineau, F., et al., Development of a PCR assay for identification of staphylococci at genus and species levels. J Clin Microbiol, 2001. 39(7): p. 2541-7.
23. Fan, S.H., et al., MpsAB is important for Staphylococcus aureus virulence and growth at atmospheric CO2 levels. Nat Commun, 2019. 10(1): p. 3627.
24. Pallen, M.J. and B.W. Wren, Bacterial pathogenomics. Nature, 2007. 449(7164): p. 835-42.
25. Hazarika, R.A., et al., Cutaneous infection associated with Staphylococcus hyicus in cattle. Res Vet Sci, 1991. 50(3): p. 374-5.
26. Foster, A.P., Staphylococcal skin disease in livestock. Vet Dermatol, 2012. 23(4): p. 342-51, e63.

## Claims

1. Oligonucleotide primer comprising:
a) the nucleotide sequence of SEQ ID NO:1 or SEQ ID NO:2; or
b) a sequence which is at least 85% identical to the nucleotide sequence of SEQ ID NO:1 or SEQ ID NO:2; or
c) the complement of (a) or (b).

2. Oligonucleotide primer according to claim 1, wherein said primer has a length of between 10-70 nucleotides, preferably between 20-50 nucleotides.

3. Oligonucleotide primer according to any of claims 1-2, wherein said primer comprises sequencing adapters.

4. Oligonucleotide primer according to any of claims 1-3, wherein said primer consists of a sequence of any of SEQ ID NOs:1-4.

5. Method for amplifying the sequence or part of the sequence of a *Staphylococcus tuf* gene, said method comprising
a) obtaining nucleic acid from bacteria of the genus *Staphylococcus*;
b) amplifying the nucleic acid with at least one oligonucleotide primer according to any of claims 1-4; and
c) optionally, sequencing the amplified nucleic acid.

6. Method according to claim 5, wherein said nucleic acid is derived from bacteria that belong to the human microbiome, preferably the human skin microbiome.

7. Method according to any of claims 5-6, wherein the nucleic acid is derived from a swab, preferably a skin swab.

8. Method according to any of claims 5-7, wherein the nucleic acid is DNA.

9. Method according to any of claims 5-8, wherein the nucleic acid is amplified in step (b) with
(i) a first primer comprising:
a) the nucleotide sequence of SEQ ID NO:1; or
b) a sequence which is at least 85% identical to the nucleotide sequence of SEQ ID NO:1; or
c) the complement of (a) or (b); and
(ii) a second primer comprising:
a) the nucleotide sequence of SEQ ID NO:2; or
b) a sequence which is at least 85% identical to the nucleotide sequence of SEQ ID NO:2; or
c) the complement of (a) or (b).

10. Method according to any of claims 5-9, wherein amplification in step (b) is effected by PCR, RT-PCR, real-time PCR or real-time RT-PCR reaction.

11. Method according to any of claims 5-10, wherein the sequencing in step (c) comprises NGS sequencing.

12. Method for identifying one or more *Staphylococcus* species and/or strains which are present in a biological sample, comprising
a) obtaining nucleic acid from said biological sample;
b) amplifying the nucleic acid with at least one oligonucleotide primer according to any of claims 1-4;
c) sequencing the amplified nucleic acid;
d) comparing the sequences obtained in step (c) with reference sequences from a plurality of *Staphylococcus* species and/or strains; and
e) assigning the sequences obtained in step (c) to a *Staphylococcus* reference sequence, thereby identifying the species and/or strains.

13. Method according to claim 12, wherein the nucleic acid is amplified in step (b) with
(i) a first primer comprising:
a) the nucleotide sequence of SEQ ID NO:1; or
b) a sequence which is at least 85% identical to the nucleotide sequence of SEQ ID NO:1; or
c) the complement of (a) or (b); and
(ii) a second primer comprising:
a) the nucleotide sequence of SEQ ID NO:2; or
b) a sequence which is at least 85% identical to the nucleotide sequence of SEQ ID NO:2; or
c) the complement of (a) or (b).

14. Use of an oligonucleotide primer comprising
a) the nucleotide sequence of SEQ ID NO:1 or SEQ ID NO:2; or
b) a sequence which is at least 85% identical to the nucleotide sequence of SEQ ID NO:1 or SEQ ID NO:2; or
c) the complement of (a) or (b)
for amplifying the sequence or part of the sequence of a *Staphylococcus tuf* gene.

15. Kit for amplifying the sequence or part of the sequence of a *Staphylococcus tuf* gene, comprising
a) at least one oligonucleotide primer of any of claims 1-5; and
b) means for performing a nucleic acid amplification reaction.


SEQUENCE LISTING

<110> Beiersdorf AG

<120> Novel oligonucleotides for detecting Staphylococcus

<130> P 109492

<160> 100

<170> BiSSAP 1.3.6

<210> 1
<211> 19
<212> DNA
<213> Artificial Sequence

<220>
<223> Forward Primer

<400> 1
acaggccgtg ttgaacgtg                                                    19

<210> 2
<211> 20
<212> DNA
<213> Artificial Sequence

<220>
<223> Reverse Primer

<400> 2
acagtacgtc caccttcacg                                                   20

<210> 3
<211> 52
<212> DNA
<213> Artificial Sequence

<220>
<223> Forward Primer with Adapter

<400> 3
tcgtcggcag cgtcagatgt gtataagaga cagacaggcc gtgttgaacg tg              52

<210> 4
<211> 54
<212> DNA
<213> Artificial Sequence

<220>
<223> Reverse Primer with Adapter

<400> 4
gtctcgtggg ctcggagatg tgtataagag acagacagta cgtccacctt cacg            54

<210> 5
<211> 467
<212> DNA
<213> Staphylococcus aureus

<220>
<223> Strain B3-17D

<400> 5
acaggccgtg ttgaacgtgg tcaaatcaaa gttggtgaag aagttgaaat catcggttta 60

catgacacat ctaaaacaac tgttacaggt gttgaaatgt tccgtaaatt attagactac 120

gctgaagctg gtgacaacat tggtgcatta ttacgtggtg ttgctcgtga agacgtacaa 180

cgtggtcaag tattagctgc tcctggttca attacaccac atactgaatt taaagcagaa 240

gtatacgtat tatcaaaaga cgaaggtgga cgtcacactc cattcttctc aaactatcgt 300

ccacaattct atttccgtac tactgacgta actggtgttg ttcacttacc agaaggtact 360

gaaatggtaa tgcctggtga taacgttgaa atgacagtag aattaatcgc tccaatcgcg 420

attgaagacg gtactcgttt ctcaatccgc gaaggtggac gtactgt 467

<210> 6
<211> 467
<212> DNA
<213> Staphylococcus aureus

<220>
<223> Strain FORC061

<400> 6
acaggccgtg ttgaacgtgg tcaaatcaaa gttggtgaag aagttgaaat catcggttta 60

catgacacat ctaaaacaac tgttacaggt gttgaaatgt tccgtaaatt attagactac 120

gctgaagctg gtgacaacat tggtgcatta ttacgtggtg ttgctcgtga agacgtacaa 180

cgtggtcaag tattagctgc tcctggttca attacaccac atactgaatt taaagcagaa 240

gtatacgtat tatcaaaaga cgaaggtgga cgtcacactc cattcttctc aaactatcgt 300

ccacaattct atttccgtac tactgacgta actggtgttg ttcacttacc agaaggaact 360

gaaatggtaa tgcctggtga taacgttgaa atgacagtag aattaatcgc tccaatcgcg 420

attgaagacg gtactcgttt ctcaatccgc gaaggtggac gtactgt 467

<210> 7
<211> 467
<212> DNA
<213> Staphylococcus aureus

<220>
<223> Strain USA300

<400> 7
acaggccgtg ttgaacgtgg tcaaatcaaa gttggtgaag aagttgaaat catcggttta 60

catgacacat ctaaaacaac tgttacaggt gttgaaatgt tccgtaaatt attagactac 120

gctgaagctg gtgacaacat tggtgcatta ttacgtggtg ttgctcgtga agacgtacaa 180

cgtggtcaag tattagctgc tcctggttca attacaccac atactgaatt caaagcagaa 240

gtatacgtat tatcaaaaga cgaaggtgga cgtcacactc cattcttctc aaactatcgt 300

ccacaattct atttccgtac tactgacgta actggtgttg ttcacttacc agaaggtact 360

gaaatggtaa tgcctggtga taacgttgaa atgacagtag aattaatcgc tccaatcgcg 420

attgaagacg gtactcgttt ctcaatccgt gaaggtggac gtactgt 467

<210> 8
<211> 467
<212> DNA
<213> Staphylococcus aureus

<220>
<223> Strain R50

<400> 8
acaggccgtg ttgaacgtgg tcaaatcaaa gttggtgaag aagttgaaat catcggttta 60

catgacacat ctaaaacaac tgttacaggt gttgaaatgt tccgtaaatt attagactac 120

gctgaagctg gtgacaacat tggtgcatta ttacgtggtg ttgctcgtga agacgtacaa 180

cgtggtcaag tattagctgc tcctggttca attacaccac atactgaatt caaagcagaa 240

gtatacgtat tatcaaaaga cgaaggcgga cgtcacactc cattcttctc aaactatcgt 300

ccacaattct atttccgtac tactgacgta actggtgttg ttcacttacc agaaggtact 360

gaaatggtaa tgcctggtga taacgttgaa atgacagtag aattaatcgc tccaatcgcg 420

attgaagacg gtactcgttt ctcaatccgt gaaggtggac gtactgt 467

<210> 9
<211> 467
<212> DNA
<213> Staphylococcus aureus

<220>
<223> Strain 55-99-44

<400> 9
acaggccgtg ttgaacgtgg tcaaatcaaa gttggtgaag aagttgaaat catcggttta 60

catgaaacat ctaaaacaac tgttacaggt gttgaaatgt tccgtaaatt attagactac 120

gctgaagctg gtgacaacat tggtgcatta ttacgtggtg ttgctcgtga agacgtacaa 180

cgtggtcaag tattagctgc tcctggttca attacaccac atactgaatt caaagcagaa 240

gtatacgtat tatcaaaaga cgaaggtgga cgtcatactc cattcttctc aaactatcgt 300

ccacaattct atttccgtac tactgacgta actggtgttg ttcacttacc agaaggtact 360

gaaatggtaa tgcctggcga taacgttgaa atgacagtag aattaatcgc tccaatcgcg 420

attgaagacg gtactcgttt ctcaatccgt gaaggtggac gtactgt 467

<210> 10
<211> 872
<212> DNA
<213> Staphylococcus auricularis

<220>
<223> Strain NCTC12101

<400> 10
acaggccgtg ttgaacgtgg tcaaatcaaa gtcggtgaag aagttgaaat catcggtatg 60

aaagacggtt cacaaaaaac aacagttact ggtgtagaaa tgttccgtaa attattagac 120

tacgctgaag ctggtgacaa catcggtgct ttattacgtg gtatttcacg tgaagaagta 180

caacgtggtc aagttttagc tgctcctggt tcaattacac cacacactaa attcactgca 240

gaagtttacg tattatctaa agatgaaggt ggacgtcaca ctccattctt ctctaactac 300

cgtccacaat tctatttccg tactactgac gtaacaggtg ttgttacttt accagaaggt 360

acagaaatgg taatgcctgg cgataacgtt aaaatggaag ttgcaggccg tgttgaacgt 420

ggtcaaatca aagtcggtga agaagttgaa atcatcggta tgaaagacgg ttcacaaaaa 480

acaacagtta ctggtgtaga aatgttccgt aaattattag actacgctga agctggtgac 540

aacatcggtg ctttattacg tggtatttca cgtgaagaag tacaacgtgg tcaagtttta 600

gctgctcctg gttcaattac accacacact aaattcactg cagaagttta cgtattatct 660

aaagatgaag gtggacgtca cactccattc ttctctaact accgtccaca attctatttc 720

cgtactactg acgtaacagg tgttgttact ttaccagaag gtacagaaat ggtaatgcct 780

ggcgataacg ttaaaatgga agttgaatta atttctccaa tcgctatcga agacggtact 840

cgtttctcaa ttcgtgaagg tggacgtact gt 872

<210> 11
<211> 467
<212> DNA
<213> Staphylococcus capitis

<220>
<223> Strain AYP1020

<400> 11
acaggccgtg ttgaacgtgg tcaaatcaaa gttggtgaag aagttgaaat catcggtatc 60

cacgaaactt ctaaaacaac tgttactggt gtagaaatgt tccgtaaatt attagactac 120

gctgaagctg gtgacaacat cggtgcttta ttacgtggtg ttgctcgtga agacgtacaa 180

cgtggtcaag tattagctgc tcctggttca atcacaccac acactaaatt caaagcggaa 240

gtttacgttt tatctaaaga cgaaggtgga cgtcacactc cattcttcag taactaccgc 300

ccacaattct atttccgtac tactgacgta actggtgttg ttaacttacc agaaggtact 360

gaaatggtta tgcctggcga caacgttgaa atgacagttg aattaatcgc tcctatcgct 420

attgaagacg gtactcgttt ctcaatccgt gaaggtggac gtactgt 467

<210> 12
<211> 467
<212> DNA
<213> Staphylococcus caprae

<220>
<223> Strain SNUC4023

<400> 12
acaggccgtg ttgaacgtgg tcaaatcaaa gttggtgaag aagttgaaat catcggtatc 60

cacgaaactt ctaaaacaac tgttactggt gtagaaatgt tccgtaaatt attagactac 120

gctgaagctg gtgacaacat cggtgcatta ttacgtggtg ttgctcgtga agacgtacaa 180

cgtggtcaag tattagctgc tcctggttca atcacaccac acactaaatt caaagctgaa 240

gtatacgttt tatctaaaga cgaaggtgga cgtcacactc cattcttcag taactaccgc 300

ccacaattct atttccgtac tactgacgta actggtgttg taaacttacc agaaggtact 360

gaaatggtta tgcctggcga caacgttgaa atgacagttg aattaatcgc tcctatcgca 420

attgaagacg gtactcgttt ctcaatccgt gaaggtggac gtactgt 467

<210> 13
<211> 467
<212> DNA
<213> Staphylococcus carnosus

<220>
<223> Strain DSM20501S

<400> 13
acaggccgtg ttgaacgtgg tcaaatcaaa gttggtgaag aagttgaaat catcggttta 60

cacgacactt ctaaaacaac tgttactggt gtagaaatgt tccgtaaatt attagactac 120

gctgaagctg gtgacaacat cggtgcatta ttacgtggtg ttgctcgtga agacgtacaa 180

cgtggtcaag tattagctgc tccaggttca atcacacctc acactaaatt caaagcagac 240

gtatacgttt tatctaaaga cgaaggtgga cgtcacactc cattcttctc aaactatcgt 300

ccacaattct atttccgtac tactgacgta actggtgttg ttaacttacc agaaggtaca 360

gaaatggtta tgcctggcga caacgttgaa atgacagtag aattaatcgc tcctatcgca 420

attgaagacg gtactcgttt ctcaatccgt gaaggtggac gtactgt 467

<210> 14
<211> 470
<212> DNA
<213> Staphylococcus carnosus

<220>
<223> Strain LTH7013

<400> 14
acaggccgtg ttgaacgtgg tcaaatcaaa gtcggtgaag aagttgaaat catcggtatc 60

actgaagaaa gcatgaaaac aacagttaca ggtgtagaaa tgttccgtaa attattagac 120

tacgctgaag ctggtgacaa cattggtgct ttattacgtg gtgttgcacg tgaagacgta 180

caacgtggac aagtattagc agctcctggc tctattactc cacacactaa atttaaagct 240

gatgtttatg ttttatctaa agatgaaggt ggacgtcata ctccattctt cactaactac 300

cgcccacaat tctacttccg tactactgac gtaactggtg ttgttaactt accagaaggt 360

actgaaatgg ttatgcctgg cgataacgta gaaatgactg ttgaattgat tgctccaatc 420

gcaatcgaag acggtactcg tttctctatc cgtgaaggtg gacgtactgt 470

<210> 15
<211> 470
<212> DNA
<213> Staphylococcus chromogenes

<220>
<223> Strain 117

<400> 15
actggccgtg ttgaacgtgg tcaaatcaaa gttggtgaag aagttgaaat catcggttta 60

tctgaagaat cttctaaaac aactgttact ggtgtagaaa tgttccgtaa gttattagac 120

tacgctgaag ctggtgacaa cattggtgct ttattacgtg gtgttgctcg tgaagacgta 180

caacgtggtc aagtattagc tgctcctggt tcaatcacac cacacacaaa atttaaagct 240

gaagtatacg ttttatcaaa agacgaaggt ggacgtcaca ctccattctt ctctaactac 300

cgcccacaat tctatttccg tactactgac gtaactggcg ttgttaactt accagaaggt 360

actgaaatgg ttatgcctgg cgataacgta gaaatggacg ttgagttaat ttctccaatc 420

gctatcgaag acggtactcg tttctcaatc cgtgaaggtg gacgtactgt 470

<210> 16
<211> 470
<212> DNA

<213> Staphylococcus chromogenes

<220>
<223> Strain SNUC1139

<400> 16
actggccgtg ttgaacgtgg tcaaatcaaa gttggtgaag aagttgaaat catcggttta 60

tctgaagaat cttctaaaac aactgttact ggtgtagaaa tgttccgtaa gttattagac 120

tacgctgaag ctggtgacaa catcggtgct ttattacgtg gtgttgctcg tgaagacgta 180

caacgtggtc aagtattagc tgctcctggt tcaatcacac cacacacaaa atttaaagct 240

gaagtatacg ttttatcaaa agacgaaggt ggacgtcaca ctccattctt ctctaactac 300

cgcccacaat tctatttccg tactactgac gtaactggcg ttgttaactt accagaaggt 360

actgaaatgg ttatgcctgg cgataacgta gaaatggacg ttgagttaat ttctccaatc 420

gctatcgaag acggtactcg tttctcaatc cgtgaaggtg gacgtactgt 470

<210> 17
<211> 470
<212> DNA
<213> Staphylococcus chromogenes

<220>
<223> Strain SNUC-126

<400> 17
actggccgtg ttgaacgtgg tcaaatcaaa gttggtgaag aagttgaaat catcggttta 60

actgaagaat cttctaaaac aactgttact ggtgtagaaa tgttccgtaa gttattagac 120

tacgctgaag ctggtgacaa cattggtgct ttattacgtg gtgttgctcg tgaagacgtt 180

caacgtggtc aagtattagc tgctcctggt tcaatcacac cacacacaaa atttaaagct 240

gaagtatacg ttttatcaaa agacgaaggt ggacgtcaca ctccattctt ctctaactac 300

cgcccacaat tctatttccg tactactgac gtaactggcg ttgttaactt accagaaggt 360

actgaaatgg ttatgcctgg cgataacgta gaaatggacg ttgagttaat ttctccaatc 420

gctatcgaag acggtactcg tttctcaatc cgtgaaggtg gacgtactgt 470

<210> 18
<211> 470
<212> DNA
<213> Staphylococcus chromogenes

<220>
<223> Strain SNUC-1379

<400> 18
actggtcgtg ttgaacgtgg tcaaatcaaa gttggtgaag aagttgaaat catcggttta 60

actgaagaat cttctaaaac aactgttact ggtgtagaaa tgttccgtaa gttattagac 120

tacgctgaag ctggtgacaa cattggtgct ttattacgtg gtgttgctcg tgaagacgta 180

caacgtggtc aagtattagc tgctcctggt tcaatcacac cacacacaaa atttaaagct 240

gaagtatacg ttttatcaaa agacgaaggt ggacgtcaca ctccattctt ctctaactac 300

cgcccacagt tctatttccg tactactgac gtaactggcg ttgttaactt accagaaggt 360

actgaaatgg ttatgcctgg cgataacgta gaaatggacg ttgagttaat ttctccaatc 420

gctatcgaag acggtactcg tttctcaatc cgtgaaggtg gacgtactgt 470

<210> 19
<211> 470
<212> DNA
<213> Staphylococcus chromogenes

<220>
<223> Strain 532

<400> 19
acaggccgtg ttgaacgtgg tcaaatcaaa gtcggtgaag aagttgaaat catcggtatg 60

caagaagaat caagcaaaac aactgttact ggtgtagaaa tgttccgtaa attattagac 120

tatgctgaag ctggtgacaa cattggtgcg ttattacgtg gtgttgcacg tgaagacatc 180

caacgtggtc aagttttagc tgctcctggt tcaattacac cacatacaaa gtttaaagcg 240

gaagtttacg ttttatctaa agatgaaggt ggacgtcata ctccattctt cagtaactac 300

cgcccacaat tctatttccg tactactgac gtaacaggcg ttgttacttt accagaaggt 360

actgaaatgg ttatgcctgg cgacaatgtt gaaatggaag ttgaactaat ttctccaatc 420

gctatcgaag acggtacacg tttctctatc cgtgaaggtg gacgtactgt 470

<210> 20
<211> 470
<212> DNA
<213> Staphylococcus cohnii

<220>
<223> Strain SNUC-3213

<400> 20
acaggccgtg ttgaacgtgg tcaaatcaaa gtcggtgaag aagttgaaat catcggtatg 60

caagaagatt caagcaaaac aactgttact ggtgtagaaa tgttccgtaa attattagac 120

tacgctgaag ctggtgacaa cattggtgcg ttattacgtg gtgttgcacg tgaagacatc 180

caacgtggtc aagttttagc tgctcctggt tcaattacac cacatacaaa ctttaaagcg 240

gaagtttacg ttttatcaaa agatgaaggt ggccgtcata cgccgttctt cagtaactac 300

cgcccacaat tctatttccg tactactgac gtaacaggtg ttgttacttt accagaaggt 360

actgaaatgg ttatgcctgg tgacaacgta gaaatggaag ttgaattaat ttctccaatc 420

gctatcgaag acggtacacg tttctctatc cgtgaaggtg gacgtactgt 470

<210> 21
<211> 470
<212> DNA
<213> Staphylococcus cohnii

<220>
<223> Strain SNUC-1120

<400> 21
acaggccgtg ttgaacgtgg tcaaatcaaa gtcggtgaag aagttgaaat catcggtatg 60

caagaagatt caagcaaaac aactgttact ggtgtagaaa tgttccgtaa attattagac 120

tacgctgaag ctggtgacaa cattggtgcg ttattacgtg gtgttgcacg tgaagacatc 180

caacgtggtc aagttatagc tgctcctggt tcaattacac cacatacaaa ctttaaagcg 240

gaagtttacg ttttatcaaa agatgaaggc ggccgtcata cgccattctt cagtaactac 300

cgcccacaat tctatttccg tactactgac gtaacaggtg ttgttacttt accagaaggt 360

actgaaatgg ttatgcctgg cgacaacgta gaaatggaag ttgaattaat ttctccaatc 420

gctatcgaag acggtacacg tttctctatc cgtgaaggtg gacgtactgt 470

<210> 22
<211> 471
<212> DNA
<213> Staphylococcus condimenti

<220>
<223> Strain DSM11674

<400> 22
acaggccgtg ttgaacgtgg tcaaatcaaa gtcggtgaag aagttgaaat catcggtatc 60

actgaagaaa gcatgaaaac aacagttaca ggtgtagaaa tgttccgtaa attattagac 120

tacgctgaag ctggtgacaa cattggtgct ttattacgtg gtgttgcacg tgaagacgta 180

caacgtggac aagtattagc agctcctggt tctattactc cacacactaa atttaaagct 240

gatgtttatg ttttatctaa agatgaaggt ggacgtcata ctccattctt cactaactac 300

cgcccacaat tctacttccg tactactgac gtaactggtg ttgttaactt accagaaggt 360

actgaaatgg ttatgcctgg cgataacgta gaaatgactg ttgaattgat ttctccaatc 420

gctatcgaag acggtactcg tttctctatc cgtgaaggtg gacgtactgt t 471

<210> 23
<211> 470
<212> DNA

<213> Staphylococcus delphini

<220>
<223> Strain DSM20771

<400> 23
actggtcgtg ttgaacgtgg tcaaatcaaa gttggtgacg aagttgaaat catcggttta 60

actgaagaat cttctaaaac aactgttact ggtgtagaaa tgttccgtaa gttattagac 120

tacgctgaag ctggtgacaa cattggtgct ttattacgtg gtgttgcacg tgaagacatc 180

aaccgtggtc aagtattagc ggctcctggt tcaatcacac cacacacaaa atttaaagcg 240

gaagtttacg tattatcaaa agacgaaggt ggtcgtcata caccattctt ctctaactac 300

cgcccacaat tctatttccg tactactgac gtaactggtg ttgtaaactt accagaaggt 360

actgaaatgg taatgcctgg cgataacgtt gaaatggaag ttgaattaat ttctccaatc 420

gctatcgaag acggtactcg tttttcaatt cgtgaaggtg gacgtactgt 470

<210> 24
<211> 470
<212> DNA
<213> Staphylococcus delphini

<220>
<223> Strain 14S03311-1

<400> 24
actggtcgtg ttgaacgtgg tcaaatcaaa gttggtgacg aagtagaaat catcggttta 60

actgaagaat cttctaaaac aactgttact ggtgtagaaa tgttccgtaa gttattagac 120

tacgctgaag ctggtgacaa cattggtgct ttattacgtg gtgttgcacg tgaagacatc 180

aaccgtggtc aagtattagc ggctcctggt tcaatcacac cacacacaaa atttaaagcg 240

gaagtttacg tattatcaaa agacgaaggt ggtcgtcata caccattctt ctctaactac 300

cgcccacaat tctatttccg tactactgac gtaactggtg ttgtaaactt accagaaggt 360

actgaaatgg taatgcctgg cgataacgtt gaaatggaag ttgaattaat ttctccaatc 420

gctatcgaag acggtactcg tttctcaatt cgtgaaggtg gacgtactgt 470

<210> 25
<211> 470
<212> DNA
<213> Staphylococcus delphini

<220>
<223> Strain 215100905101-2

<400> 25
actggtcgtg ttgaacgtgg tcaaatcaaa gttggtgacg aagttgaaat catcggttta 60

actgaagaat cttctaaaac aactgttact ggtgtagaaa tgttccgtaa gttattagac      120

tacgctgaag ctggtgacaa cattggtgct ttattacgtg gtgttgcacg tgaagacatc      180

aaccgtggtc aagtattagc ggctcctggt tcaatcacac cacacacaaa atttaaagcg      240

gaagtttacg tattatcaaa agacgaaggt ggtcgtcata caccattctt ctctaactac      300

cgcccacaat tctatttccg tactactgac gtaactggtg ttgtaaactt accagaaggt      360

actgaaatgg taatgcctgg cgataacgtt gaaatggaag ttgaattaat ttctccaatc      420

gctatcgaag acggtactcg tttctcaatt cgtgaaggtg gacgtactgt                 470

<210> 26
<211> 467
<212> DNA
<213> Staphylococcus devriesei

<220>
<223> Strain SNUC-1409

<400> 26
acaggccgtg ttgaacgtgg tcaaatcaaa gttggtgaag aagttgaaat catcggatta       60

cacgacactt ctaaaacaac tgttactggt gtagaaatgt tccgtaaatt attagactat      120

gctgaagctg gtgacaacat cggtgcatta ttacgtggtg ttgctcgtga agacgtacaa      180

cgtggacaag tattagctgc tccaggttca attactcctc atacaaaatt caaagctgac      240

gtatacgttt tatctaaaga cgaaggtgga cgtcacactc cattcttctc aaactatcgt      300

ccacaattct atttccgtac tactgacgta actggtgtag ttaacttacc agaaggtact      360

gaaatggtta tgcctggcga caacgttgaa atgacagtag aattaatcgc tcctatcgct      420

attgaagacg gtactcgttt ctcaatccgt gaaggtggac gtactgt                    467

<210> 27
<211> 467
<212> DNA
<213> Staphylococcus devriesei

<220>
<223> Strain SNUC-1156

<400> 27
acaggccgtg ttgaacgtgg tcaaatcaaa gttggtgaag aagttgaaat catcggatta       60

cacgagactt ctaaaacaac tgttactggt gtagaaatgt tccgtaaatt attagactat      120

gctgaagctg gtgacaacat cggtgcatta ttacgtggtg ttgctcgtga agacgtacaa      180

cgtggacaag tattagctgc tccaggttca attactcctc atacaaaatt caaagctgac      240

gtatacgttt tatctaaaga cgaaggtgga cgtcacactc cattcttctc aaactatcgt      300

ccacaattct atttccgtac tactgacgta actggtgtag ttaacttacc agaaggtact      360

gaaatggtta tgcctggcga caacgttgaa atgacagtag aattaatcgc tcctatcgct 420

attgaagacg gtactcgttt ctcaatccgt gaaggtggac gtactgt 467

<210> 28
<211> 470
<212> DNA
<213> Staphylococcus edaphicus

<220>
<223> Strain CCM-8730

<400> 28
acaggccgtg ttgaacgtgg tcaaatcaaa gtcggtgaag aagttgaaat cattggtatg 60

caagaagatt caagcaaaac aactgttact ggtgtagaaa tgttccgtaa attattagac 120

tacgctgaag ctggtgacaa cattggtgca ttattacgtg gtgtttcacg tgatgacgta 180

caacgtggtc aagttttagc tgctcctggt actattacac cacatacaaa attcaaagcg 240

gatgtttacg ttttatctaa agatgaaggt ggccgtcata caccattctt cactaactac 300

cgcccacaat tctatttccg tactactgac gtaactggtg ttgttaactt accagaaggt 360

actgaaatgg ttatgcctgg cgataacgtt gaaatggaag ttgaattaat ttctccaatc 420

gctattgaag acggtacacg tttctctatc cgtgaaggtg gacgtactgt 470

<210> 29
<211> 467
<212> DNA
<213> Staphylococcus epidermidis

<220>
<223> Strain ATCC-12228

<400> 29
acaggccgtg ttgaacgtgg tcaaatcaaa gttggtgaag aagttgaaat catcggtatg 60

cacgaaactt ctaaaacaac tgttactggt gtagaaatgt tccgtaaatt attagactac 120

gctgaagctg gtgacaacat cggtgcttta ttacgtggtg ttgcacgtga agacgtacaa 180

cgtggtcaag tattagctgc tcctggttct attacaccac acacaaaatt caaagctgaa 240

gtatacgtat tatctaaaga tgaaggtgga cgtcacactc cattcttcac taactatcgc 300

ccacaattct atttccgtac tactgacgta actggtgttg taaacttacc agaaggtaca 360

gaaatggtta tgcctggcga caacgttgaa atgacagttg aattaatcgc tccaatcgct 420

atcgaagacg gaactcgttt ctcaattcgt gaaggtggac gtactgt 467

<210> 30
<211> 467
<212> DNA

<213> Staphylococcus epidermidis

<220>
<223> Strain SE95

<400> 30
acaggccgtg ttgaacgtgg tcaaatcaaa gttggtgaag aagttgaaat catcggtatg 60

cacgaaactt ctaaaacaac tgttactggt gtagaaatgt tccgtaaatt attagactac 120

gctgaagctg gtgacaacat cggtgcttta ttacgtggtg ttgcacgtga agacgtacaa 180

cgtggtcaag tattagctgc tcctggttct attacaccac acacaaaatt caaagctgaa 240

gtatacgtat tatctaaaga tgaaggtgga cgtcacactc cattcttcac taactatcgc 300

ccacaattct atttccgtac tactgacgta actggtgttg taaacttacc agaaggtaca 360

gaaatggtta tgcctggcga taacgttgaa atgacagttg aattaatcgc tccaatcgct 420

atcgaagacg gaactcgttt ctcaattcgt gaaggtggac gtactgt 467

<210> 31
<211> 467
<212> DNA
<213> Staphylococcus epidermidis

<220>
<223> SEI

<400> 31
acaggccgtg ttgaacgtgg tcaaatcaaa gttggtgaag aagttgaaat catcggtatg 60

cacgaaactt ctaaaacaac tgttactggt gtagaaatgt tccgtaaatt attagactac 120

gctgaagctg gtgacaacat cggtgcttta ttacgtggtg ttgcacgtga agacgtacaa 180

cgtggtcaag tattagctgc tcctggttct attacaccac acacaaaatt caaagctgaa 240

gtatacgtat tatctaaaga tgaaggtgga cgtcacactc cattcttcac taactatcgc 300

ccacaattct atttccgtac tactgacgta actggtgttg taaacttacc agaaggtaca 360

gaaatggtta tgcctggtga caacgttgaa atgacagttg aattaatcgc tccaatcgct 420

atcgaagacg gaactcgttt ctcaattcgt gaaggtggac gtactgt 467

<210> 32
<211> 467
<212> DNA
<213> Staphylococcus epidermidis

<220>
<223> Strain FDAARGOS-529

<400> 32
acaggccgtg ttgaacgtgg tcaaatcaaa gttggtgaag aagttgaaat catcggtatg 60

catgaaactt ctaaaacaac tgttactggt gtagaaatgt tccgtaagtt attagactac 120

gctgaagctg gtgacaacat cggtgcttta ttacgtggtg ttgcacgtga agacgtacaa 180

cgtggtcaag tattagctgc tcctggttct attacaccac acacaaaatt caaagctgaa 240

gtatacgtat tatctaaaga tgaaggtgga cgtcacactc cattcttcac taactatcgc 300

ccacaattct atttccgtac tactgacgta actggtgttg taaacttacc agaaggtaca 360

gaaatggtta tgcctggcga caacgttgaa atgacagttg aattaatcgc tccaatcgct 420

atcgaagacg gaactcgttt ctcaattcgt gaaggtggac gtactgt 467

<210> 33
<211> 470
<212> DNA
<213> Staphylococcus equorum

<220>
<223> Strain 900-4

<400> 33
acaggccgtg ttgaacgtgg tcaaatcaaa gtcggtgctg aaattgaaat catcggtatg 60

caagaggaat caactaagac aactgttaca ggtgttgaaa tgttccgtaa attactagac 120

tacgctgaag ctggcgacaa cattggtgct ttattacgtg gtgtatcacg tgatgacatc 180

tctcgtggac aagttttagc tgcgcctggt actattacac cgcatactaa cttcaaagca 240

gacgtttatg ttttatcaaa agaagaaggt ggacgtcata ctccattctt cactaactac 300

cgtccgcaat tctatttccg tactactgac gtaacaggcg ttgttacttt accagaaggt 360

actgaaatgg ttatgcctgg cgataacgtt gaaatgcaag ttgaattgat ttcaccaatc 420

gctatcgaag atggtactcg tttctctatc cgtgaaggtg gacgtactgt 470

<210> 34
<211> 470
<212> DNA
<213> Staphylococcus equorum

<220>
<223> Strain SNUC-284

<400> 34
acaggccgtg ttgaacgtgg tcaaatcaaa gtcggtgctg aaattgaaat catcggtatg 60

caagaggaat caactaagac aactgttaca ggtgttgaaa tgttccgtaa attactagac 120

tacgctgaag ctggcgacaa cattggtgct ttattacgtg gtgtatcacg tgatgacatt 180

tctcgtggac aagttttagc tgcgcctggt actattacac cgcatactaa cttcaaagca 240

gacgtttatg ttttatcaaa agaagaaggt ggacgtcata ctccattctt cactaactac 300

cgtccgcaat tctatttccg tactactgac gtaacaggcg ttgttacttt accagaaggt 360

actgaaatgg ttatgcctgg cgataacgtt gaaatgcaag ttgaattgat ttcaccaatc 420

gctatcgaag atggtactcg tttctctatc cgtgaaggtg gacgtactgt 470

<210> 35
<211> 470
<212> DNA
<213> Staphylococcus equorum

<220>
<223> Strain BC9

<400> 35
acaggccgtg ttgaacgtgg tcaaatcaaa gtcggcgctg aaattgaaat catcggtatg 60

caagaggaat caactaaaac aactgttaca ggtgttgaaa tgttccgtaa attactagac 120

tacgctgaag ctggcgacaa cattggtgct ttattacgtg gtgtatcacg tgatgacatc 180

tctcgtggac aagttttagc tgcacctggt actattacac cgcatactaa cttcaaagca 240

gacgtttatg ttttatcaaa agaagaaggt ggacgtcata ctccattctt cactaactac 300

cgtccgcaat tctatttccg tactactgac gtaacaggtg ttgttacttt accagaaggt 360

actgaaatgg ttatgcctgg cgataacgtt gaaatgcaag ttgaattgat ttcaccaatc 420

gctatcgaag atggtactcg tttctctatc cgtgaaggtg gacgtactgt 470

<210> 36
<211> 470
<212> DNA
<213> Staphylococcus equorum

<220>
<223> Strain SNUC-476

<400> 36
acaggccgtg ttgaacgtgg tcaaatcaaa gtcggtgctg aaattgaaat catcggtatg 60

caagaggaat caactaaaac aactgttaca ggtgttgaaa tgttccgtaa attattagac 120

tacgctgaag ctggcgacaa cattggtgct ttattacgtg gtgtatcacg tgatgacatc 180

tctcgtggac aagttttagc tgcgcctggt actattacac cgcatactaa cttcaaagca 240

gacgtttatg ttttatcaaa agaagaaggt ggacgtcata ctccattctt cactaactac 300

cgtccgcaat tctatttccg tactactgac gtaactggcg ttgttagttt accagaaggt 360

actgaaatgg ttatgcctgg cgataacgtt gaaatgcaag ttgaattgat ttcaccaatc 420

gctatcgaag atggtactcg tttctctatc cgtgaaggtg gacgtactgt 470

<210> 37
<211> 470
<212> DNA

<213> Staphylococcus felis

<220>
<223> Strain F1-k127-55

<400> 37
acaggccgtg ttgaacgtgg tcaaatcaaa gttggtgaag aagttgaaat catcggttta 60

actgaacaat cttctaaaac aactgttact ggtgtagaaa tgttccgtaa gttattagac 120

tacgctgaag ctggtgacaa catcggtgca ttattacgtg gtgttgctcg tgaagacatc 180

aaccgtggac aagttttagc tgctcctggt tcaatcacac cacacacaaa atttaaagca 240

gaagtttacg tattatcaaa agacgaaggt ggacgtcaca cgccattctt ctctaactac 300

cgcccacaat tctatttccg tactactgac gtaactggtg tagttaactt accagaaggt 360

acagaaatgg ttatgcctgg cgataacgtt gaaatggaag ttgaattaat ttctccaatc 420

gctatcgaag acggtacacg tttctcaatc cgtgaaggtg gacgtactgt 470

<210> 38
<211> 470
<212> DNA
<213> Staphylococcus felis

<220>
<223> Strain F27-k127-298

<400> 38
acaggccgtg ttgaacgtgg tcaaatcaaa gttggtgaag aagttgaaat catcggttta 60

actgaacaat cttctaaaac aactgttact ggtgtagaaa tgttccgtaa gttattagac 120

tacgctgaag ctggtgacaa catcggtgca ttattacgtg gtgttgctcg tgaagacatc 180

aaccgtggac aagttttagc tgctcctggt tcaatcacac cacacacaaa atttaaagct 240

gaagtttacg tattatcaaa agacgaaggt ggacgtcaca cgccattctt ctctaactac 300

cgcccacaat tctatttccg tactactgac gtaactggtg tagttaactt accagaaggt 360

acagaaatgg ttatgcctgg cgataacgtt gaaatggaag ttgaattaat ttctccaatc 420

gctatcgaag acggtacacg tttctcaatc cgtgaaggtg gacgtactgt 470

<210> 39
<211> 470
<212> DNA
<213> Staphylococcus felis

<220>
<223> Strain F8-k127-533

<400> 39
acaggccgtg ttgaacgtgg tcaaatcaaa gttggtgaag aagttgaaat catcggttta 60

actgaacaat cttctaaaac aactgttact ggtgtagaaa tgttccgtaa gttattagac 120

tacgctgaag ctggtgacaa catcggtgca ttattacgtg gtgttgctcg tgaagacatc 180

aaccgtggac aagttttagc tgctcctggt tcaatcacac cacacacaaa atttaaagct 240

gaagtttacg tattatcaaa agatgaaggt ggacgtcata cgccattctt ctctaactac 300

cgcccacaat tctatttccg tactactgac gtaactggtg tagttaactt accagaaggt 360

acagaaatgg ttatgcctgg cgataacgtt gaaatggaag ttgaattaat ttctccaatc 420

gctatcgaag acggtacacg tttctcaatc cgtgaaggtg gacgtactgt 470

<210> 40
<211> 470
<212> DNA
<213> Staphylococcus felis

<220>
<223> Strain F29-k127-272

<400> 40
acaggccgtg ttgaacgtgg tcaaatcaaa gttggtgaag aagttgaaat catcggttta 60

actgaacaat cttctaaaac aactgttact ggtgtagaaa tgttccgtaa gttattagac 120

tacgctgaag ctggtgacaa catcggtgca ttattacgtg gtgttgctcg tgaagacatc 180

aaccgtggac aagttttagc tgctcctggt tcaatcacac cacacacaaa atttaaagct 240

gaagtttacg tattatcaaa agacgaaggt ggacgtcata cgccattctt ctctaactac 300

cgcccacaat tctatttccg tactactgac gtaactggtg tagttaactt accagaaggt 360

acagaaatgg ttatgcctgg cgataacgtt gaaatggaag ttgaattaat ttctccaatc 420

gctatcgaag acggtacacg tttctcaatc cgtgaaggtg gacgtactgt 470

<210> 41
<211> 470
<212> DNA
<213> Staphylococcus fleurettii

<220>
<223> Strain DSM-13212

<400> 41
actggtcgtg ttgaacgtgg tcaaatcaaa gttggtgaag aagttgaaat catcggttta 60

gctgaagaat cttctaaaac aactgttact ggtgtagaaa tgttccgtaa attattagac 120

tacgctgaag ctggtgacaa cattggtgct ttattacgtg gtgttgcacg tgaagacatc 180

aaccgtggtc aagtattagc tgctcctggt tcaattacac cacacacaaa gtttaaagca 240

gaagtttacg tattatctaa agacgaaggt ggtcgtcaca caccattctt ctctaactac 300

cgcccacaat tctatttccg tactactgac gtaactggcg ttgttcactt accagaaggt 360

actgaaatgg ttatgcctgg cgataacgtt gaaatggaag ttgaattaat ttcaccaatc 420

gctatcgaag acggtactcg tttctcaatt cgtgaaggtg gacgtactgt 470

<210> 42
<211> 470
<212> DNA
<213> Staphylococcus fleurettii

<220>
<223> Strain NCTC13829

<400> 42
acaggccgtg ttgaacgtgg tcaaatcaaa gttggtgaag agattgaaat catcggttta 60

actgaagaat cttctaaaac aactgtaact ggtgttgaaa tgttccgtaa attattagac 120

ttcgctgaag ctggagataa catcggtgct ttattacgtg gtgttgctcg tgaagacgtt 180

aaccgtggtc aagtattagc taaaccaggt tcaatcactc ctcacactaa attcaaagcg 240

gaagtttatg tattatcaaa agaagaaggt ggacgtcata ctccattctt cactaactac 300

cgcccacaat tctatttccg tacaactgac gtaactggtg tagtaaactt accagaaggc 360

attgaaatgg ttatgcctgg cgacaacgtt gaaatggacg ttgaattaat ttcaccaatc 420

gctattgaag acggtactcg tttctcaatt cgtgaaggtg gacgtacagt 470

<210> 43
<211> 470
<212> DNA
<213> Staphylococcus gallinarum

<220>
<223> Strain DSM20610

<400> 43
acaggccgtg ttgaacgtgg tcaaatcaaa gtcggtgaag aaatcgaaat catcggtatg 60

caagaagaat caagcaaaac aactgttact ggtgtagaaa tgttccgtaa gttattagac 120

tacgctgaag ctggtgacaa cattggtgca ttattacgtg gtgtttcacg tgaagatgtt 180

caacgtggtc aagttttagc tgctcctggt actattacac cacatacaaa attcaaagcg 240

gaagtttacg ttttatctaa agacgaaggt ggacgtcata ctccattctt cactaactac 300

cgcccacaat tctatttccg tactactgac gtaactggtg ttgttaactt accagaaggt 360

actgaaatgg ttatgcctgg cgataacgtt gaaatgatcg ttgaattaat ttctccaatc 420

gctatcgaag acggtactcg tttctctatc cgtgaaggtg gacgtactgt 470

<210> 44
<211> 470
<212> DNA

<213> Staphylococcus gallinarum

<220>
<223> Strain SNUC2913

<400> 44
acaggccgtg ttgaacgtgg tcaaatcaaa gtcggtgaag aaatcgaaat catcggtatg 60

caagaagaat caagcaaaac aactgttact ggtgtagaaa tgttccgtaa gttattagac 120

tacgctgaag ctggtgacaa cattggtgca ttattacgtg gtgtttcacg tgaagatgtt 180

caacgtggtc aagttttagc tgctcctggt actattacac cacatacaaa attcaaagcg 240

gaagtttacg ttttatctaa agacgaaggt ggacgtcata ctccattctt cactaactac 300

cgcccacaat tctatttccg tactactgac gtaactggtg ttgttaactt accagaaggt 360

actgaaatgg ttatgcctgg cgataacgtt gaaatggtcg ttgaattaat ttctccaatc 420

gctatcgaag acggtactcg tttctctatc cgtgaaggtg gacgtactgt 470

<210> 45
<211> 467
<212> DNA
<213> Staphylococcus haemolyticus

<220>
<223> Strain 7067

<400> 45
acaggccgtg ttgaacgtgg tcaaatcaaa gttggtgaag aagttgaaat cattggtatc 60

catgacactt ctaaaacaac tgttactggt gtagaaatgt tccgtaaatt attagactac 120

gctgaagctg gtgacaacat cggtgcatta ttacgtggtg ttgctcgtga agacgtacaa 180

cgtggtcaag tattagctgc tccaggttca atcacacctc acacaaaatt caaagcagac 240

gtatacgttt tatctaaaga cgaaggtgga cgtcacactc cattcttcac aaactatcgt 300

ccacaattct atttccgtac tactgacgta actggtgttg ttaacttacc agaaggtaca 360

gaaatggtta tgcctggcga caacgttgaa atgacagtag aattaatcgc tcctatcgcg 420

attgaagacg gtactcgttt ctcaatccgt gaaggtggac gtactgt 467

<210> 46
<211> 467
<212> DNA
<213> Staphylococcus haemolyticus

<220>
<223> Strain DE0499

<400> 46
acaggccgtg ttgaacgtgg tcaaatcaaa gttggtgaag aagttgaaat cattggtatc 60

catgatactt ctaaaacaac tgttactggt gtagaaatgt tccgtaaatt attagactac 120

gctgaagctg gtgacaacat cggtgcatta ttacgtggtg ttgctcgtga agacgtacaa 180

cgtggtcaag tattagctgc tccaggttca atcacaccac acacaaaatt caaagcagac 240

gtatacgttt tatctaaaga cgaaggtgga cgtcacactc cattcttcac aaactatcgt 300

ccacaattct atttccgtac tactgacgta actggtgttg ttaacttacc agaaggtaca 360

gaaatggtta tgcctggcga caacgttgaa atgacagtag aattaatcgc tcctatcgcg 420

attgaagacg gtactcgttt ctcaatccgt gaaggtggac gtactgt 467

<210> 47
<211> 467
<212> DNA
<213> Staphylococcus haemolyticus

<220>
<223> Strain DE0081

<400> 47
acaggccgtg ttgaacgtgg tcaaatcaaa gttggtgaag aagttgaaat cattggtatc 60

catgacactt ctaaaacaac tgttactggt gtagaaatgt tccgtaaatt attagactac 120

gctgaagctg gtgacaacat cggtgcatta ttacgtggtg ttgctcgtga agacgtacaa 180

cgtggtcaag tattagctgc tccaggttca atcacaccac acacaaaatt caaagcagac 240

gtatacgttt tatctaaaga cgaaggtgga cgtcacactc cattcttcac aaactatcgt 300

ccacaattct atttccgtac tactgacgta actggtgttg ttaacttacc agaaggtaca 360

gaaatggtta tgcctggcga caacgttgaa atgacagtag aattaatcgc tcctatcgcg 420

attgaagacg gtactcgttt ctcaatccgt gaaggtggac gtactgt 467

<210> 48
<211> 467
<212> DNA
<213> Staphylococcus haemolyticus

<220>
<223> Strain SDA2

<400> 48
acaggccgtg ttgaacgtgg tcaaatcaaa gttggtgaag aagttgaaat cattggtatc 60

catgacactt ctaaaacaac tgttactggt gtagaaatgt tccgtaaatt attagactac 120

gctgaagctg gtgacaacat cggtgcatta ttacgtggtg ttgctcgtga agacgtacaa 180

cgtggtcaag tattagctgc tccaggttca atcacacctc acacaaaatt taaagcagac 240

gtatacgttt tatctaaaga cgaaggtgga cgtcacactc cattcttcac aaactatcgt 300

ccacaattct atttccgtac tactgacgta actggtgttg ttaacttacc agaaggtaca 360

gaaatggtta tgcctggcga caacgttgaa atgacagtag aattaatcgc tcctatcgcg 420

attgaagacg gtactcgttt ctcaatccgt gaaggtggac gtactgt 467

<210> 49
<211> 467
<212> DNA
<213> Staphylococcus haemolyticus

<220>
<223> Strain SNUC119

<400> 49
acaggccgtg ttgaacgtgg tcaaatcaaa gttggtgaag aagttgaaat cattggtatc 60

catgacactt ctaaaacaac tgttactggt gtagaaatgt tccgtaaatt attagactac 120

gctgaagctg gtgacaacat cggtgcatta ttacgtggtg ttgctcgtga agacgtacaa 180

cgtggtcaag tattagctgc tccaggttca atcacacctc acacaaaatt caaagcagac 240

gtatacgttt tatctaaaga cgaaggtgga cgtcacactc cattcttcac aaactatcgt 300

ccacaattct atttccgtac tactgacgta actggtgttg ttaacttacc agaaggtact 360

gaaatggtta tgcctggcga caacgttgaa atgacagtag aattaatcgc tcctatcgcg 420

attgaagacg gtactcgttt ctcaatccgt gaaggtggac gtactgt 467

<210> 50
<211> 467
<212> DNA
<213> Staphylococcus haemolyticus

<220>
<223> Strain 7068

<400> 50
acaggccgtg ttgaacgtgg tcaaatcaaa gttggtgaag aagttgaaat cattggtatc 60

catgacactt ctaaaacaac tgttactggt gtagaaatgt tccgtaaatt attagactac 120

gctgaagctg gtgacaacat cggtgcatta ttacgtggtg ttgctcgtga agacgtacaa 180

cgtggtcaag tattagctgc tccaggttca atcacacctc acacaaaatt taaagcagac 240

gtatacgttt tatctaaaga cgaaggtgga cgtcacactc cattcttcac aaactatcgt 300

ccacaattct atttccgtac tactgacgta actggtgttg ttaacttacc agaaggtact 360

gaaatggtta tgcctggcga caacgttgaa atgacagtag aattaatcgc tcctatcgcg 420

attgaagacg gtactcgttt ctcaatccgt gaaggtggac gtactgt 467

<210> 51
<211> 468
<212> DNA

<213> Staphylococcus hominis

<220>
<223> Strain 19A

<400> 51
acaggccgtg ttgaacgtgg tcaaatcaaa gttggtgaag aagttgaaat tattggtatc 60

aaagaaactt ctaaaacaac tgttactggt gtagaaatgt tccgtaaatt attagactac 120

gctgaagctg gtgacaacat cggtgcttta ttacgtggtg ttgctcgtga agatgtacaa 180

cgtggtcaag tattagctgc tccaggttca attacacctc acacaaaatt caaagcagac 240

gtatacgttt tatcaaaaga tgaaggtgga cgtcatactc cattcttcac taactatcgt 300

ccacaattct atttccgtac tactgacgta actggtgttg ttaacttacc agaaggtact 360

gaaatggtaa tgcctggcga caacgttgaa atgacaggta gaattaatcg ctcctatcgc 420

gattgaagac ggtactcgtt tctcaatccg tgaaggtgga cgtactgt 468

<210> 52
<211> 467
<212> DNA
<213> Staphylococcus hominis

<220>
<223> Strain J6

<400> 52
acaggccgtg ttgaacgtgg tcaaatcaaa gttggtgaag aagttgaaat tattggtatc 60

aaagaaactt ctaaaacaac tgttactggt gtagaaatgt tccgtaaatt attagactac 120

gctgaagctg gtgacaacat cggtgcttta ttacgtggtg ttgctcgtga agatgtacaa 180

cgtggtcaag tattagctgc tccaggttca attacacctc acacaaaatt caaagcagac 240

gtatacgttt tatcaaaaga tgaaggtgga cgtcatactc cattcttctc taactatcgt 300

ccacaattct atttccgtac tactgacgta actggtgttg ttaacttacc agaaggtact 360

gaaatggtaa tgcctggtga caacgttgaa atgacagtag aattaatcgc tcctatcgcg 420

attgaagacg gtactcgttt ctcaatccgt gaaggtggac gtactgt 467

<210> 53
<211> 467
<212> DNA
<213> Staphylococcus hominis

<220>
<223> Strain K1

<400> 53
acaggccgtg ttgaacgtgg tcaaatcaaa gttggtgaag aagttgaaat tattggtatc 60

aaagaaactt ctaaaacaac tgttactggt gtagaaatgt tccgtaaatt attagactat 120

gctgaagctg gtgacaacat cggtgcttta ttacgtggtg ttgctcgtga agatgtacaa 180

cgtggtcaag tattagctgc tccaggttca attacacctc acacaaaatt caaagcagac 240

gtatacgttt tatcaaaaga tgaaggtggt cgtcatactc cattcttcac taactatcgt 300

ccacaattct atttccgtac tactgacgta actggtgttg ttaacttacc agaaggtact 360

gaaatggtaa tgcctggcga caacgttgaa atgacagtag aattaatcgc tcctatcgcg 420

attgaagacg gtactcgttt ctcaatccgt gaaggtggac gtactgt 467

<210> 54
<211> 470
<212> DNA
<213> Staphylococcus hyicus

<220>
<223> Strain NCTC7944

<400> 54
acaggccgtg ttgaacgtgg tcaaatcaaa gtcggtgaag aagttgaaat catcggtatc 60

actgaagaaa gcaagaaaac aacagttaca ggtgtagaaa tgttccgtaa attattagac 120

tacgctgaag ctggtgacaa catcggtgct ttattacgtg gtgttgcacg tgaagacgta 180

caacgtggac aagtattagc agctcctggc tctattactc cacacacaaa attcaaagct 240

gatgtttacg ttttatctaa agaagaaggt ggacgtcata ctccattctt cactaactac 300

cgcccacaat tctacttccg tactactgac gtaactggcg ttgttcactt accagaaggt 360

actgaaatgg ttatgcctgg cgataacgta gaaatgactg ttgaattgat cgctccaatc 420

gcgattgaag acggtactcg tttctctatc cgtgaaggtg gacgtactgt 470

<210> 55
<211> 470
<212> DNA
<213> Staphylococcus intermedius

<220>
<223> Strain NCTC11048

<400> 55
actggccgtg ttgaacgtgg tcaaatcaaa gttggtgacg aagttgaaat catcggttta 60

actgaagaat cttctaaaac aactgttact ggtgtagaaa tgttccgtaa gttattagac 120

tacgctgaag ctggtgacaa cattggtgct ttattacgtg gtgttgcacg tgaagacatc 180

aaccgtggtc aagtattagc agctcctggt tcaatcacac cacacacaaa atttaaagcg 240

gaagtttacg tattatcaaa agacgaaggt ggacgtcata caccattctt ctctaactac 300

cgcccacaat tctatttccg tactactgac gtaactggtg ttgtaaactt accagaaggt 360

actgaaatgg taatgcctgg cgataacgtt gaaatggaag ttgaattaat ttctccaatc 420

gctatcgaag acggtacacg tttctcaatt cgtgaaggtg gacgtactgt 470

<210> 56
<211> 470
<212> DNA
<213> Staphylococcus kloosii

<220>
<223> Strain SNUC4696

<400> 56
acaggccgtg ttgaacgtgg tcaaatcaaa gtcggtgaag aagttgaaat catcggtatg 60

caagatgaat caagcaaaac aacagttact ggtgtagaaa tgttccgtaa attattagat 120

tacgctgaag ctggtgacaa cattggtgct ttattacgtg gtgttgcacg tgaagacatc 180

caacgtggtc aagttttagc tgctcctggt tcaattacac cacacactaa attcaaagca 240

gaagtttatg tattatcaaa agacgaaggt ggacgtcata ctccattctt cagtaactac 300

cgcccacaat tctatttccg tactactgac gtaacaggtg ttgttacttt accagaaggt 360

actgaaatgg ttatgcctgg tgacaacgtt gaaatggacg ttgaattaat ttcaccaatc 420

gctatcgaag acggtactcg tttctcaatt cgtgaaggtg gccgtactgt 470

<210> 57
<211> 467
<212> DNA
<213> Staphylococcus lugdunensis

<220>
<223> NCTC12217

<400> 57
acaggccgtg ttgaacgtgg tcaaatcaaa gtcggtgaag aagttgaaat tattggtatc 60

cacgatacta ctaaaacaac tgttactggt gtagaaatgt tccgtaaatt attagactac 120

gctgaagctg gtgacaacat cggtgcgtta ttacgtggtg ttgctcgtga agatgtacaa 180

cgtggacaag tattagctgc tccaggttca attacacctc acactaaatt taaagctgac 240

gtatatgttt tatctaaaga tgaaggtgga cgtcatacac cattcttctc aaactaccgc 300

ccacaattct atttccgtac tacagacgta actggtgttg ttaacttacc agaaggtaca 360

gaaatggtta tgcctggcga caacgttgaa atgacagttg aattaatcgc tccaatcgct 420

atcgaagacg gaactcgttt ctcaattcgt gaaggtggac gtactgt 467

<210> 58
<211> 467
<212> DNA

<213> Staphylococcus lugdunensis

<220>
<223> Strain NCTC12217

<400> 58
acaggccgtg ttgaacgtgg tcaaatcaaa gtcggtgaag aagttgaaat tattggtatc 60

cacgatacta ctaaaacaac tgttactggt gtagaaatgt tccgtaaatt attagactac 120

gctgaagctg gtgacaacat cggtgcgtta ttacgtggtg ttgctcgtga agatgtacaa 180

cgtggacaag tattagctgc tccaggttca attacacctc acactaaatt taaagctgac 240

gtatatgttt tatctaaaga tgaaggtgga cgtcatacac cattcttctc aaactaccgc 300

ccacaattct atttccgtac tacagacgta actggtgttg ttaacttacc agaaggtaca 360

gaaatggtta tgcctggcga caacgttgaa atgacagttg aattaatcgc tccaatcgct 420

atcgaagacg gaactcgttt ctcaattcgt gaaggtggac gtactgt 467

<210> 59
<211> 467
<212> DNA
<213> Staphylococcus lugdunensis

<220>
<223> Strain VISLISI_33

<400> 59
acaggccgtg ttgaacgtgg tcaaatcaaa gtcggtgaag aagttgaaat tattggtatc 60

cacgatacta ctaaaacaac ggttactggt gtagaaatgt tccgtaaatt attagactat 120

gctgaagctg gtgacaacat cggtgcgtta ttacgtggtg ttgctcgtga agatgtacaa 180

cgtggacaag tattagctgc tccaggttca attacacctc acactaaatt taaagctgac 240

gtatatgttt tatctaaaga tgaaggtgga cgtcatacac cattcttctc aaactaccgc 300

ccacaattct atttccgtac tacagacgta actggtgttg ttaacttacc agaaggtaca 360

gaaatggtta tgcctggtga caacgttgaa atgacagttg aattaatcgc tccaatcgct 420

atcgaagacg gaactcgttt ctcaattcgt gaaggtggac gtactgt 467

<210> 60
<211> 470
<212> DNA
<213> Staphylococcus lutrae

<220>
<223> Strain ATCC700373

<400> 60
acaggccgtg ttgaacgtgg tcaaatcaaa gttggtgacg aagtagaaat catcggttta 60

actgaagaat cttctaaaac aactgttact ggtgtagaaa tgttccgtaa gttattagac 120

tacgctgaag ctggagacaa cattggtgct ttattacgtg gtgttgcacg tgaagacatc 180

aaccgtggtc aagttttagc agcacctggt tcaatcacac cacatacaaa atttaaagcg 240

gaagtttacg tattatcaaa agacgaaggt ggtcgtcata caccattctt ctctaactac 300

cgcccacaat tctatttccg tacaactgac gtaacaggtg ttgttaattt accagaaggt 360

actgaaatgg taatgcctgg tgacaacgtt gaaatggaag ttgaattaat ttcaccaatc 420

gctatcgaag acggtacacg tttctcaatc cgtgaaggtg gacgtactgt 470

<210> 61
<211> 470
<212> DNA
<213> Staphylococcus massiliensis

<220>
<223> Strain S46

<400> 61
acaggccgtg ttgaacgtgg tcaaatcaaa gtcggtgaag aagttgaaat catcggtctt 60

gctgaagaat caagcaaaac aactgttaca ggtgtagaaa tgttccgtaa gttattagac 120

tacgctgaag ctggtgacaa tatcggtgct ttattacgtg gtgttgcacg tgaagacgtt 180

aaccgtggtc aagtattagc tgctcctggt tcaatcacac ctcacactaa attcaaatca 240

gaagtatacg tattatcaaa agacgaaggt ggtcgtcata caccattctt ctctaactac 300

cgtccacaat tctatttccg tactactgac gtaactggtg ttatccaatt accagaaggt 360

acagaaatgg ttatgcctgg cgataacgtt gaaatggaag ttgaattaat ttctaaaatc 420

gctatcgaag acggtactcg tttctcaatc cgtgaaggtg gacgtactgt 470

<210> 62
<211> 470
<212> DNA
<213> Staphylococcus nepalensis

<220>
<223> Strain JS9

<400> 62
acaggccgtg ttgaacgtgg tcaaatcaaa gtcggtgaag aagttgaaat catcggtatg 60

caagaagaat caagcaaaac aactgttact ggtgtagaaa tgttccgtaa attattagac 120

tatgctgaag ctggtgacaa cattggtgca ttattacgtg gtgttgcacg tgaagatatc 180

caacgtggtc aagttttagc tgctcctggt tcaattacac cgcacacaaa cttcaaagca 240

gaagtttatg tcttatctaa agatgaaggt ggacgtcata cgccattctt cagtaactac 300

cgcccacaat tctatttccg tactactgat gtaactggtg ttgttacttt accagaaggt 360

actgaaatgg ttatgcctgg cgataacgta gaaatgcaag ttgaattgat ttctccaatc 420

gctattgaag acggtactcg tttctcaatc cgtgaaggtg gacgtactgt 470

<210> 63
<211> 467
<212> DNA
<213> Staphylococcus pasteuri

<220>
<223> Strain JS7

<400> 63
acaggccgtg ttgaacgtgg tcaaatcaaa gttggtgaag aagttgaaat catcggttta 60

catgacactt ctaaaacaac tgttactggt gtagaaatgt tccgtaaatt attagactac 120

gctgaagctg gtgacaacat cggtgcatta ttacgtggtg ttgctcgtga agacgtacaa 180

cgtggacaag tattagctgc tccaggttca atcacacctc atacaaaatt caaagcggaa 240

gtttacgttt tatctaaaga tgaaggtgga cgtcatactc cattcttcag taactaccgc 300

ccacaattct atttccgtac tactgacgta actggtgtag ttaacttacc agaaggtact 360

gaaatggtta tgcctggtga caacgttgaa atgacagttg aattaatcgc tccaatcgcg 420

attgaagacg gtactcgttt ctcaatccgt gaaggtggac gtactgt 467

<210> 64
<211> 467
<212> DNA
<213> Staphylococcus pasteuri

<220>
<223> Strain 3C

<400> 64
acaggccgtg ttgaacgtgg tcaaatcaaa gttggtgaag aagttgaaat catcggttta 60

catgacactt ctaaaacaac tgttactggt gtagaaatgt tccgtaaatt attagactac 120

gctgaagctg gtgacaacat cggtgcatta ttacgtggtg tcgctcgtga agacgtacaa 180

cgtggacaag tattagctgc tccaggttca atcacacctc atacaaaatt caaagcggaa 240

gtttacgttt tatctaaaga tgaaggtgga cgtcatactc cattcttcag taactaccgc 300

ccacaattct atttccgtac tactgacgta actggtgtag ttaacttacc agaaggtact 360

gaaatggtta tgcctggtga caacgttgaa atgacagttg aattaatcgc tccaatcgcg 420

attgaagacg gtactcgttt ctcaatccgt gaaggtggac gtactgt 467

<210> 65
<211> 467
<212> DNA

<213> Staphylococcus petrasii

<220>
<223> Strain CCUG62657

<400> 65
acaggccgtg ttgaacgtgg tcaaatcaaa gttggtgaag aagttgaaat catcggttta 60

cacgacactt ctaaaacaac tgttacaggt gtagaaatgt tccgtaagtt attagactac 120

gctgaagctg gtgacaacat cggtgcttta ttacgtggta tcgctcgtga agacgtacaa 180

cgtggtcaag tattagctgc tccaggttca atcacacctc acactaaatt caaagcagac 240

gtatacgttt tatctaaaga cgaaggtgga cgtcacactc cattcttctc aaactatcgt 300

ccacaattct atttccgtac tactgacgta actggtgttg ttaacttacc agaaggtaca 360

gaaatggtta tgcctggcga caacgttgaa atgacagtag aattaatcgc tcctatcgcg 420

attgaagacg gtactcgttt ctcaatccgt gaaggtggac gtactgt 467

<210> 66
<211> 467
<212> DNA
<213> Staphylococcus petrasii

<220>
<223> Strain CCM8421

<400> 66
acaggccgtg ttgaacgtgg tcaaatcaaa gttggtgaag aagttgaaat tattggtatc 60

catgatactt ctaaaacaac tgttacaggt gtagaaatgt tccgtaaatt attagactac 120

gctgaagctg gtgacaacat cggtgcatta ttacgtggtg ttgctcgtga agacgtacaa 180

cgtggtcaag tattagctgc tccaggttca atcacacctc acactaaatt caaagcagac 240

gtatacgttt tatctaaaga cgaaggtgga cgtcacactc cattcttctc aaactatcgt 300

ccacaattct atttccgtac tactgacgta actggtgttg ttaacttacc agaaggtaca 360

gaaatggtta tgcctggcga caacgttgaa atgacagtag aattaatcgc tcctatcgct 420

attgaagacg gtactcgttt ctcaatccgt gaaggtggac gtactgt 467

<210> 67
<211> 470
<212> DNA
<213> Staphylococcus pettenkoferi

<220>
<223> Strain FDAARGOS288

<400> 67
acaggccgtg ttgaacgtgg tcaaatcaaa gttggtgaag aagttgaaat catcggtatg 60

gctgatgaat cacaaaaaac aactgttact ggtgtagaaa tgttccgtaa gttattagac 120

tacgctgaag ctggtgacaa catcggtgcg ttattacgtg gtattgcacg tgaagacgtt 180

caacgtggtc aagtattagc tgctcctggt tcaatcacac cacacactaa attcaaagct 240

gaagtttacg tattatctaa agacgaaggt ggacgtcaca ctccattctt ctctaactac 300

cgtccacaat tctatttccg tactactgac gtaactggtg tagttaactt accagaaggt 360

acagaaatgg ttatgcctgg tgacaacgtt gaaatggacg ttgaattaat ttcaccaatc 420

gctatcgaag acggtactcg tttctctatc cgtgaaggtg gacgtactgt 470

<210> 68
<211> 470
<212> DNA
<213> Staphylococcus piscifermentans

<220>
<223> Strain NCTC13836

<400> 68
acaggccgtg ttgaacgtgg tcaaatcaaa gtcggtgaag aagttgaaat catcggtatc 60

actgaagaaa gcatgaaaac tacagttaca ggtgtagaaa tgttccgtaa attattagac 120

tacgctgaag ctggtgacaa cattggtgct ttattacgtg gtgttgcacg tgaagacgta 180

caacgtggac aagtattagc agctcctggc tctattactc cacacactaa atttaaagct 240

gatgtttacg ttttatctaa agacgaaggt ggacgtcaca ctccattctt ctctaactac 300

cgcccacaat tctacttccg tactactgac gtaacaggcg ttgttaactt accagaaggt 360

actgaaatgg ttatgcctgg cgataacgta gaaatgactg ttgaattgat tgctccaatc 420

gcaatcgaag acggtactcg tttctctatc cgtgaaggtg gacgtactgt 470

<210> 69
<211> 470
<212> DNA
<213> Staphylococcus pseudintermedius

<220>
<223> Strain 53-60

<400> 69
actggtcgtg ttgaacgtgg tcaaatcaaa gttggtgacg aagtagaaat catcggttta 60

actgaagaat cttctaaaac aactgttact ggtgtagaaa tgttccgtaa gttattagac 120

tacgctgaag ctggtgacaa cattggtgct ttattacgtg gtgttgcacg tgaagacatc 180

aaccgtggtc aagtattagc tgctcctggt tcaatcacac cacacacaaa atttaaagcg 240

gaagtttacg tattatcaaa agacgaaggt ggtcgtcata caccattctt ctctaactac 300

cgcccacaat tctatttccg tactactgac gtaactggtg ttgtaaactt accagaaggt 360

actgaaatgg taatgcctgg cgataacgtt gaaatggaag ttgaattaat ttctccaatc 420

gctatcgaag acggtactcg tttctcaatt cgtgaaggtg gacgtactgt 470

<210> 70
<211> 470
<212> DNA
<213> Staphylococcus pseudintermedius

<220>
<223> Strain 49-44

<400> 70
actggtcgtg ttgaacgtgg tcaaatcaaa gttggtgacg aagtagaaat catcggttta 60

actgaagaat cttctaaaac aactgttact ggtgtagaaa tgttccgtaa gttattagac 120

tatgctgaag ctggtgacaa cattggtgct ttattacgtg gtgttgcacg tgaagacatc 180

aaccgtggtc aagtattagc tgctcctggt tcaatcacac cacacacaaa atttaaagcg 240

gaagtttacg tattatcaaa agacgaaggt ggtcgtcata caccattctt ctctaactac 300

cgcccacaat tctatttccg tactactgac gtaactggtg ttgtaaactt accagaaggt 360

actgaaatgg taatgcctgg cgataacgtt gaaatggaag ttgaattaat ttctccaatc 420

gctatcgaag acggtactcg tttctcaatt cgtgaaggtg gacgtactgt 470

<210> 71
<211> 470
<212> DNA
<213> Staphylococcus pseudintermedius

<220>
<223> Strain 063228

<400> 71
actggtcgtg ttgaacgtgg tcaaatcaaa gttggtgacg aagtagaaat catcggttta 60

actgaagaat cttctaaaac aactgttact ggtgtagaaa tgttccgtaa gttattagac 120

tacgctgaag ctggtgacaa cattggtgct ttattacgtg gtgttgcacg tgaagacatc 180

aaccgtggtc aagtattagc tgctcctggt tcaatcacac cacacacaaa atttaaagcg 240

gaagtttacg tattatcaaa agacgaaggt ggtcgtcata caccattctt ctctaactac 300

cgcccacaat tctatttccg tactactgac gtaactggtg ttgtaaactt accagaaggt 360

actgaaatgg taatgcctgg cgataacgtt gaaatggaag ttgaattaat ttctccaatc 420

gctattgaag acggtactcg tttctcaatt cgtgaaggtg gacgtactgt 470

<210> 72
<211> 470
<212> DNA

<213> Staphylococcus rostri

<220>
<223> Strain DSM21968

<400> 72
actggccgtg ttgaacgtgg tcaaatcaaa gttggtgaag aagttgaaat catcggttta 60

actgatgaat cagctaaaac aactgttact ggtgtagaaa tgttccgtaa gttattagac 120

tacgctgaag ctggtgacaa cattggtgca ttattacgtg gtgtttcacg tgaagatgta 180

caacgtggtc aagtattagc agctccaggt tctattacac cacacacaaa atttaaagcg 240

gaagtttacg tattatcaaa agacgaaggt ggacgtcaca ctcctttctt ctctaactac 300

cgcccacaat tctatttccg tactactgac gtaactggtg ttgttaactt accagaaggt 360

actgaaatgg ttatgcctgg cgataacgtt gaaatggaag ttgaattaat ttctccaatc 420

gctatcgaag acggtactcg tttctcaatc cgtgaaggtg gacgtactgt 470

<210> 73
<211> 467
<212> DNA
<213> Staphylococcus saccharolyticus

<220>
<223> Strain 12B0021-3c

<400> 73
acaggccgtg ttgaacgtgg tcaaatcaaa gttggtgaag aagttgaaat catcggtatg 60

catgatacaa ctaaaacaac tgttactggt gtagaaatgt tccgtaaatt attagattac 120

gctgaagctg gtgataacat cggtgctcta ttacgtggta ttgcacgtga agacgttcaa 180

cgtgggcaag tattagctgc acctggttca atcacaccac acactaaatt caaagctgaa 240

gtttacgttt tatctaaaga tgaaggtgga cgtcatactc cattcttcag taactatcgt 300

ccacaattct atttccgtac tactgacgta actggtgttg ttaacttacc agaaggcact 360

gaaatggtta tgcctggtga caacgttgaa atgacagttg aattaatcgc tcctattgct 420

atcgaagacg gaactcgttt ctcaattcgt gaaggtggac gtactgt 467

<210> 74
<211> 467
<212> DNA
<213> Staphylococcus saccharolyticus

<220>
<223> Strain DVP2-17-2406-1c

<400> 74
acaggccgtg ttgaacgtgg tcaaatcaaa gttggtgaag aagttgaaat catcggtatg 60

catgatacaa ctaaaacaac tgttactggt gtagaaatgt tccgtaaatt attagattac 120

gctgaagctg gtgataacat cggtgcttta ttacgtggta ttgcacgtga agacgttcaa 180

cgtggtcaag tattagctgc acctggttca atcacaccac acactaaatt caaagctgaa 240

gtttacgttt tatctaaaga tgaaggtgga cgtcatactc cattcttcag taactatcgt 300

ccacaattct atttccgtac tactgacgta actggtgttg ttaacttacc agaaggcact 360

gaaatggtta tgcctggtga caatgttgaa atgacagttg aattaatcgc tcctatcgct 420

atcgaagacg gaactcgttt ctcaattcgt gaaggtggac gtactgt 467

<210> 75
<211> 470
<212> DNA
<213> Staphylococcus saprophyticus

<220>
<223> Strain 1A

<400> 75
acaggccgtg ttgaacgtgg tcaaatcaaa gtcggtgaag aaatcgaaat catcggtatg 60

caagaagaat caagcaaaac aactgttact ggtgtagaaa tgttccgtaa attattagac 120

tacgctgaag ctggtgacaa cattggtgca ttattacgtg gtgtttcacg tgatgacgta 180

caacgtggtc aagttttagc tgctcctggt actattacac cacatacaaa attcaaagcg 240

gatgtttacg ttttatctaa agatgaaggt ggtcgtcata caccattctt cactaactac 300

cgcccacaat tctatttccg tactactgac gtaactggtg ttgttaactt accagaaggt 360

actgaaatgg ttatgcctgg tgataacgtt gaaatggatg ttgaattaat ttctccaatc 420

gctattgaag acggtactcg tttctctatc cgtgaaggtg gacgtactgt 470

<210> 76
<211> 470
<212> DNA
<213> Staphylococcus saprophyticus

<220>
<223> Strain FDAARGOS-137

<400> 76
acaggccgtg ttgaacgtgg tcaaatcaaa gtcggtgaag aaatcgaaat catcggtatg 60

caagaagaat caagcaaaac aactgttact ggtgtagaaa tgttccgtaa attattagac 120

tacgctgaag ctggtgacaa cattggtgca ttattacgtg gtgtttcacg tgatgacgta 180

caacgtggtc aagttttagc tgctcctggt actattacac cacatacaaa attcaaagcg 240

gatgtttacg ttttatctaa agatgaaggt ggtcgtcata caccattctt cactaactac 300

cgcccacaat tctatttccg tactactgac gtaactggtg ttgttaactt accagaaggt 360

actgaaatgg ttatgcctgg cgataacgtt gaaatggatg ttgaattaat ttctccaatc 420

gctattgaag acggtactcg tttctctatc cgtgaaggtg gacgtactgt 470

<210> 77
<211> 470
<212> DNA
<213> Staphylococcus saprophyticus

<220>
<223> Strain ATCC15305

<400> 77
acaggccgtg ttgaacgtgg tcaaatcaaa gtcggtgaag aaatcgaaat catcggtatg 60

caagaagaat caagcaaaac aactgttact ggtgtagaaa tgttccgtaa attattagac 120

tacgctgaag ctggtgacaa cattggtgca ttattacgtg gtgtttcacg tgatgatgta 180

caacgtggtc aagttttagc tgctcctggt actatcacac cacatacaaa attcaaagcg 240

gatgtttacg ttttatctaa agatgaaggt ggtcgtcata cgccattctt cactaactac 300

cgcccacaat tctatttccg tactactgac gtaactggtg ttgttaactt accagaaggt 360

actgaaatgg ttatgcctgg cgataacgtt gaaatggatg ttgaattaat ttctccaatc 420

gctattgaag acggtactcg tttctctatc cgtgaaggtg gacgtactgt 470

<210> 78
<211> 470
<212> DNA
<213> Staphylococcus schleiferi

<220>
<223> Strain OT1-1

<400> 78
actggtcgtg ttgaacgtgg tcaaatcaaa gttggtgaag aagttgaaat catcggttta 60

gctgaagaat cttctaaaac aactgttact ggtgtagaaa tgttccgtaa attactagac 120

tacgctgaag ctggtgacaa cattggtgct ttattacgtg gtgttgcacg tgaagacatc 180

aaccgtggtc aagtattagc tgctcctggt tcaattacac cacacacaaa gtttaaagca 240

gaagtttacg tattatctaa agacgaaggt ggtcgtcaca ctccattctt ctctaactac 300

cgcccacaat tctatttccg tactactgac gtaactggcg ttgttcactt accagaaggt 360

actgaaatgg ttatgcctgg cgataacgtt gaaatggaag ttgaattaat ttcaccaatc 420

gctatcgaag acggtacgcg tttctcaatt cgtgaaggtg gacgtactgt 470

<210> 79
<211> 467
<212> DNA

<213> Staphylococcus schweitzeri

<220>
<223> >S-schweitzeri-NCTC13712

<400> 79
acaggccgtg ttgaacgtgg tcaaatcaaa gttggtgaag aagttgaaat catcggttta 60

catgacacat ctaaaacaac tgttacaggt gttgaaatgt tccgtaaatt attagactac 120

gctgaagctg gtgacaacat cggtgcatta ttacgtggtg ttgctcgtga agacgtacaa 180

cgtggtcaag tattagctgc tcctggttca attacaccac atactgaatt caaagcagaa 240

gtttatgtat tatcaaaaga cgaaggtgga cgtcacactc cattcttctc aaactatcgt 300

ccacaattct atttccgtac tactgacgta actggtgttg tacacttacc agaaggtact 360

gaaatggtaa tgcctggtga taacgttgaa atgaaagtag aattaatcgc tcctatcgcg 420

attgaagacg gtactcgttt ctcaatccgt gaaggtggac gtactgt 467

<210> 80
<211> 428
<212> DNA
<213> Staphylococcus sciuri

<220>
<223> Strain B9-58B

<400> 80
acaggccgtg ttgaacgtgg tcaaatcact gttggtgaag aagttgaaat catcggttta 60

actgaagaat cttctaaaac aactgtaact ggtgttgaaa tgttccgtaa attattagac 120

ttcgctgaag ctggagataa catcggtgca ttattacgtg gtgttgctcg tgaagacgtt 180

aaccgtggtc aagtattagc taaaccaggt tcaatcacac ctcacactaa attcaaagct 240

gaagtttatg tattatctaa agacgaaggt ggacgtcata ctccattctt cacaaactac 300

cgcccacaat tctatttccg tactactgac gtaactggtg tagttaactt accagaaggt 360

actgaaatgg ttatgcctgg cgacaacgtt gaaatggacg ttgaattaat ttcaccaatc 420

gctattga 428

<210> 81
<211> 467
<212> DNA
<213> Staphylococcus simiae

<220>
<223> Strain NCTC13838

<400> 81
acaggccgtg ttgaacgtgg tcaaatcaaa gttggtgaag aagttgaaat catcggatta 60

cacgaaactt ctaaaacaac tgttactggt gtagaaatgt tccgtaaatt attggactac 120

gctgaagctg gtgacaacat tggtgcatta ttacgtggtg ttgctcgtga agacgtacaa 180

cgtggtcaag tattagctgc tcctggttca atcacaccac acactaactt caaagctgaa 240

gtttatgtat tatcaaaaga cgaaggtgga cgtcatactc cattcttctc aaactatcgt 300

ccacaattct atttccgtac tactgacgta actggtgttg ttaatttacc agaaggtact 360

gaaatggtaa tgcctggtga taacgttgaa atgacagtag aattaatcgc tcctatcgcg 420

attgaagacg gtactcgttt ctcaatccgt gaaggtggac gtactgt 467

<210> 82
<211> 470
<212> DNA
<213> Staphylococcus simulans

<220>
<223> Strain MR1

<400> 82
acaggccgtg ttgaacgtgg tcaaatcaaa gtcggtgaag aagttgaaat catcggtatc 60

actgaagaaa gcaagaaaac aacagttaca ggtgtagaaa tgttccgtaa attattagac 120

tacgctgaag ctggtgacaa catcggtgct ttattacgtg gtgttgcacg tgaagacgta 180

caacgtggac aagtattagc agctcctggc tctattactc cacacacaaa attcaaagct 240

gatgtttacg ttttatctaa agaagaaggt ggacgtcata ctccattctt cactaactac 300

cgcccacaat tctacttccg tactactgac gtaactggcg ttgttcactt accagaaggt 360

actgaaatgg ttatgcctgg cgataacgta gaaatgactg ttgaattaat cgctccaatc 420

gcgattgaag acggtactcg tttctctatc cgtgaaggtg gacgtactgt 470

<210> 83
<211> 471
<212> DNA
<213> Staphylococcus stepanovicii

<220>
<223> Strain NCTC13839

<400> 83
actggccgtg ttgaacgtgg acaagttaaa gtcggtgaag aaatcgaaat catcggttta 60

actgaagaat catctaaaac tactgtaact ggtgtagaaa tgttccgtaa attattagac 120

ttcgctgaag ctggagataa tatcggtgca ttattacgtg gtgttgctcg tgaagacgtt 180

aaccgtggtc aagtattagc taaaccaggt tcaatcacac ctcacactaa atttaaagca 240

gaagtttatg tattatctaa agatgaaggt ggacgtcata ctccattctt cactaactac 300

cgcccacaat tctatttccg tactactgac gtaactggtg tagttaactt accagaaggt 360

actgaaatgg ttatgcctgg cgataacgtt gaaatggacg ttgaattaat ttcaccaatc 420

gctattgaag acggtactcg tttctcaatc cgtgaaggtg gacgtacagt t 471

<210> 84
<211> 470
<212> DNA
<213> Staphylococcus succinus

<220>
<223> Strain 14BME20

<400> 84
acaggccgtg ttgaacgtgg tcaaatcaaa gtcggtgaag aaatcgaaat tatcggtatc 60

actgaagaat caagtaaaac aactgttact ggtgtagaaa tgttccgtaa attactagac 120

tacgctgaag ctggtgacaa cattggtgcg ttattacgtg gggtttcacg tgacgatgta 180

caacgtggtc aagttttagc tgctcctggt actattacac cacatacaaa attcaaatct 240

gaagtttacg ttttatcaaa agatgaaggt ggacgtcata ctccattctt cactaactac 300

cgcccacaat tctatttccg tactactgat gtaactggtg ttgtaagttt accagaaggt 360

actgaaatgg ttatgcctgg cgataacgtt gaaatggaag ttgaattaat ctcaccaatc 420

gctattgaag atggtactcg tttctctatc cgtgagggtg gacgtactgt 470

<210> 85
<211> 467
<212> DNA
<213> Staphylococcus warneri

<220>
<223> Strain 16A

<400> 85
acaggccgtg ttgaacgtgg tcaaatcaaa gttggtgaag aagttgaaat catcggttta 60

catgacactt ctaaaacaac tgttactggt gtagaaatgt tccgtaagtt attagactac 120

gctgaagctg gtgacaacat cggtgcttta ttacgtggtg ttgctcgtga agacgtacaa 180

cgtggtcaag tattagctgc tcctggttca attacaccac atacaaaatt caaagcggaa 240

gtttacgttt tatctaaaga cgaaggtgga cgtcacactc cattcttcag taactaccgc 300

ccacaattct atttccgtac tactgacgta actggcgttg ttcaattacc agaaggtact 360

gaaatggtta tgcctggtga taacgttgaa atgacagtag aattaatcgc tcctatcgcg 420

attgaagacg gtactcgttt ctcaatccgt gaaggtggac gtactgt 467

<210> 86
<211> 470
<212> DNA

<213> Staphylococcus xylosus

<220>
<223> Strain S170

<400> 86
acaggccgtg ttgaacgtgg tcaaatcaaa gtcggtgaag aaatcgaaat catcggtatg 60

caagaagagt caagcaaaac aacagttact ggtgtagaaa tgttccgtaa attattagac 120

tacgctgaag ctggtgacaa cattggtgca ttattacgtg gtgtttcacg tgatgatatc 180

caacgtggtc aagttttagc tgctcctggt actattacac cacatactaa attcaaagct 240

gacgtttatg ttttatcaaa agatgaaggt ggtcgtcata caccattctt cactaactac 300

cgcccacaat tctatttccg tactactgac gtaacaggtg ttgttacttt accagaaggt 360

actgaaatgg ttatgcctgg cgataacgta gaaatgaacg ttgaattgat ttctccaatc 420

gctattgaag acggtactcg tttctctatc cgtgaaggtg gacgtactgt 470

<210> 87
<211> 470
<212> DNA
<213> Staphylococcus xylosus

<220>
<223> Strain 2

<400> 87
acaggccgtg ttgaacgtgg tcaaatcaaa gtcggtgaag aaatcgaaat cattggtatg 60

caagaagagt caagcaaaac aacagttact ggtgtagaaa tgttccgtaa attattagac 120

tacgctgaag ctggtgacaa cattggtgca ttattacgtg gtgtttcacg tgatgacatc 180

caacgtggtc aagttttagc tgctcctggt actattacac cacatactaa attcaaagct 240

gacgtttatg ttttatcaaa agatgaaggt ggtcgtcata caccattctt cactaactac 300

cgcccacaat tctatttccg tactactgac gtaacaggtg ttgttacttt accagaaggt 360

actgaaatgg ttatgcctgg cgataacgta gaaatgaacg ttgaattgat ttctccaatc 420

gctattgaag acggtactcg tttctctatc cgtgaaggtg gacgtactgt 470

<210> 88
<211> 467
<212> DNA
<213> Staphylococcus epidermidis

<220>
<223> Strain N1

<400> 88
acaggccgtg ttgaacgtgg tcaaatcaaa gttggtgaag aagttgaaat catcggtatg 60

cacgaaactt ctaaaacaac tgttactggt gtagaaatgt tccgtaaatt attagactac 120

gctgaagctg gtgacaacat cggtgcttta ttacgtggtg ttgcacgtga agacgtacaa 180

cgtggtcaag tattagctgc tcctggttct attacaccac acacaaaatt caaagctgaa 240

gtatacgtat tatctaaaga tgaaggtgga cgtcacactc cattcttcac taactatcgc 300

ccacaattct atttccgtac tactgacgta actggtgttg taaacttacc agaaggtaca 360

gaaatggtta tgcctggcga caacgttgaa atgacagttg aattaatcgc tccaatcgct 420

attgaagacg gaactcgttt ctcaattcgt gaaggtggac gtactgt 467

<210> 89
<211> 467
<212> DNA
<213> Staphylococcus epidermidis

<220>
<223> Strain N2

<400> 89
acaggccgtg ttgaacgtgg tcaaatcaaa gttggtgaag aagttgaaat catcggtatg 60

catgaaactt ctaaaacaac tgttactggt gtagaaatgt tccgtaaatt attagactac 120

gctgaagctg gtgacaacat cggtgcttta ttacgtggtg ttgcacgtga agacgtacaa 180

cgtggtcaag tattagctgc tcctggttct attacaccac acacaaaatt caaagctgaa 240

gtatacgtat tatctaaaga tgaaggtgga cgtcacactc cattcttcac taactatcgc 300

ccacaattct atttccgtac tactgacgta actggtgttg taaacttacc agaaggtaca 360

gaaatggtta tgcctggcga caacgttgaa atgacagttg aattaatcgc tccaatcgct 420

atcgaagacg gaactcgttt ctcaattcgt gaaggtggac gtactgt 467

<210> 90
<211> 467
<212> DNA
<213> Staphylococcus epidermidis

<220>
<223> Strain N3

<400> 90
acaggccgtg ttgaacgtgg tcaaatcaaa gttggtgaag aagttgaaat catcggtatg 60

cacgaaactt ctaaaacaac tgttactggt gtagaaatgt tccgtaaatt attagactac 120

gctgaagctg gtgacaacat cggtgcttta ttacgtggtg ttgcacgtga agacgtacaa 180

cgtggtcaag tattagctgc tcctggttct attacaccac acacaaaatt caaagctgaa 240

gtatacgtat tatctaaaga tgaaggtgga cgtcacactc cattcttcac taactatcgc 300

ccacaattct atttccgtac tactgacgta actggtgttg taaacctacc agaaggtaca 360

gaaatggtta tgcctggcga caacgttgaa atgacagttg aattaatcgc tccaatcgct 420

atcgaagacg gaactcgttt ctcaattcgt gaaggtggac gtactgt 467

<210> 91
<211> 467
<212> DNA
<213> Staphylococcus capitis

<220>
<223> Strain N1

<400> 91
acaggccgtg ttgaacgtgg tcaaatcaaa gttggtgaag aagttgaaat catcggtatc 60

cacgaaactt ctaaaacaac tgttactggt gtagaaatgt tccgtaaatt attagactac 120

gctgaagctg gtgacaacat cggtgcttta ttacgtggtg ttgctcgtga agacgtacaa 180

cgtggtcaag tattagctgc tcctggttca atcacaccac acactaaatt caaagcggaa 240

gtttacgttt tatctaaaga cgaaggtgga cgtcacactc cattcttcag taactaccga 300

ccacaattct atttccgtac tactgacgta actggtgttg ttaacttacc agaaggtact 360

gaaatggtta tgcctggcga caacgttgaa atgacagttg aattaatcgc tcctatcgct 420

attgaagacg gtactcgttt ctcaatccgt gaaggtggac gtactgt 467

<210> 92
<211> 467
<212> DNA
<213> Staphylococcus haemolyticus

<220>
<223> Strain N1

<400> 92
acaggccgtg ttgaacgtgg tcaaatcaaa gttggtgaag aagttgaaat cattggtatc 60

catgacactt ctaaaacaac tgttactggt gtagaaatgt tccgtaaatt attagactac 120

gctgaagctg gtgacaacat tggtgcatta ttacgtggtg ttgctcgtga agacgtacaa 180

cgtggtcaag tattagctgc tccaggttca atcacacctc acacaaaatt taaagcagac 240

gtatacgttt tatctaaaga cgaaggtgga cgtcacactc cattcttcac aaactatcgt 300

ccacaattct atttccgtac tactgacgta actggtgttg ttaacttacc agaaggtaca 360

gaaatggtta tgcctggtga caacgttgaa atgacagtag aattaatcgc tcctatcgcg 420

attgaagacg gtactcgttt ctcaatccgt gaaggtggac gtactgt 467

<210> 93
<211> 467
<212> DNA

<213> Staphylococcus hominis

<220>
<223> Strain N1

<400> 93
acaggccgtg ttgaacgtgg tcaaatcaaa gttggtgaag aagttgaaat tattggtatc 60

aaagaaactt ctaaaacaac tgttactggt gtagaaatgt tccgtaaatt attagactac 120

gctgaagctg gtgacaacat cggtgcttta ttacgtggtg ttgctcgtga agatgtacaa 180

cgtggtcaag tattagctgc tccaggttca attacacctc acacaaaatt caaagcagac 240

gtatacgttt tatcaaaaga tgaaggtgga cgtcatactc cattcttcac taactatcgt 300

ccacaattct atttccgtac tactgacgta actggtgttg ttaacttacc agaaggtact 360

gaaatggtaa tgcctggcga caacgttgaa atgacagtag aattaatcgc tcctatcgcg 420

attgaagacg gtactcgttt ctcaatccgt gaaggtggac gtactgt 467

<210> 94
<211> 467
<212> DNA
<213> Staphylococcus hominis

<220>
<223> Strain N2

<400> 94
acaggccgtg ttgaacgtgg tcaaatcaaa gttggtgaag aagttgaaat tattggtatc 60

aaagaaactt ctaaaacaac tgttactggt gtagaaatgt tccgtaaatt attagactac 120

gctgaagctg gtgacaacat cggtgcttta ttacgtggtg ttgctcgtga agatgtacaa 180

cgtggtcaag tattagctgc tccaggttca attacacctc acacaaaatt caaagcagac 240

gtatacgttt tatcaaaaga tgaaggtgga cgtcatactc cattcttcac taactatcgt 300

ccacaattct atttccgtac tactgacgta actggtgttg ttaacttacc agaaggtact 360

gaaatggtaa tgcctggcga taacgttgaa atgacagtag aattaatcgc tcctatcgcg 420

attgaagacg gtactcgttt ctcaatccgt gaaggtggac gtactgt 467

<210> 95
<211> 467
<212> DNA
<213> Staphylococcus hominis

<220>
<223> Strain N3

<400> 95
acaggccgtg ttgaacgtgg tcaaatcaaa gttggtgaag aagttgaaat tattggtatc 60

aaagaaactt ctaaaacaac tgttactggt gtagaaatgt tccgcaaatt attagactac 120

gctgaagctg gtgacaacat cggtgcttta ttacgtggtg ttgctcgtga agatgtacaa 180

cgtggtcaag tattagctgc tccaggttca attacacctc acacaaaatt caaagcagac 240

gtatacgttt tatcaaaaga tgaaggtgga cgtcatactc cattcttcac taactatcgt 300

ccacaattct atttccgtac tactgacgta actggtgttg ttaacttacc agaaggtact 360

gaaatggtaa tgcctggcga taacgttgaa atgacagtag aattaatcgc tcctatcgcg 420

attgaagacg gtactcgttt ctcaatccgt gaaggtggac gtactgt 467

<210> 96
<211> 467
<212> DNA
<213> Staphylococcus hominis

<220>
<223> Strain N4

<400> 96
acaggccgtg ttgaacgtgg tcaaatcaaa gttggtgaag aagttgaaat tattggtatc 60

aaagaaactt ctaaaacaac tgttactggt gtagaaatgt tccgtaaatt attagactac 120

gctgaagctg gtgacaacat cggtgcttta ttacgtggtg ttgctcgtga agatgtacaa 180

cgtggtcaag tattagctgc tccaggttca attacacctc acacaaaatt caaagcagac 240

gtatacgttt tatcaaaaga tgaaggtggt cgtcatactc cattcttcac taactatcgt 300

ccacaattct atttccgtac tactgacgta actggtgttg ttaacttacc agaaggtact 360

gaaatggtaa tgcctggcga caacgttgaa atgacagtag aattaatcgc tcctatcgcg 420

attgaagacg gtactcgttt ctcaatccgt gaaggtggac gtactgt 467

<210> 97
<211> 467
<212> DNA
<213> Staphylococcus hominis

<220>
<223> Strain N5

<400> 97
acaggccgtg ttgaacgtgg tcaaatcaaa gttggtgaag aagttgaaat tattggtatc 60

aaagaaactt ctaaaacaac tgttactggt gtagaaatgt tccgtaaatt attagactac 120

gctgaagctg gtgacaacat cggtgcttta ttacgtggtg ttgctcgtga agatgtacaa 180

cgtggtcaag tattagctgc tccaggttca attacacctc acacaaaatt caaagcagac 240

gtatatgttt tatcaaaaga tgaaggtgga cgtcatactc cattcttcac taactatcgt 300

ccacaattct atttccgtac tactgacgta actggtgttg ttaacttacc agaaggtact 360

gaaatggtaa tgcctggcga caacgttgaa atgacagtag aattaatcgc tcctatcgcg 420

attgaagacg gtactcgttt ctcaatccgt gaaggtggac gtactgt 467

<210> 98
<211> 467
<212> DNA
<213> Staphylococcus warneri

<220>
<223> Strain N1

<400> 98
acaggccgtg ttgaacgtgg tcaaatcaaa gttggtgaag aagttgaaat catcggttta 60

catgacactt ctaaaacaac tgttactggt gtagaaatgt tccgtaagtt attagactac 120

gctgaagctg gtgacaacat cggtgcttta ttacgtggtg ttgctcgtga agacgtacaa 180

cgtggtcaag tattagctgc tcctggttca attacaccac atacaaaatt caaagcggaa 240

gtttacgttt tatctaaaga cgaaggtgga cgtcacactc cattcttcag taactaccgc 300

ccacaattct atttccgtac tactgacgta actggcgttg ttcaattacc agaaggtact 360

gaaatggtta tgcctggtga caacgttgaa atgacagtag aattaatcgc tcctatcgcg 420

attgaagacg gtactcgttt ctcaatccgt gaaggtggac gtactgt 467

<210> 99
<211> 467
<212> DNA
<213> Staphylococcus warneri

<220>
<223> Strain N2

<400> 99
acaggccgtg ttgaacgtgg tcaaatcaaa gttggtgaag aagttgaaat catcggttta 60

catgacactt ctaaaacaac tgttactggt gtagaaatgt tccgtaagtt attagactac 120

gctgaagctg gtgacaacat cggtgcttta ttacgtggtg ttgctcgtga agacgtacaa 180

cgtggtcaag tattagctgc tcctggttca attacaccac atacaaaatt caaagcggaa 240

gtttacgttt tatctaaaga cgaaggtgga cgtcacactc cattcttcag taactaccgc 300

ccgcaattct atttccgtac tactgacgta actggcgttg ttcaattacc agaaggtact 360

gaaatggtta tgcctggtga caacgttgaa atgacagtag aattaatcgc tcctatcgcg 420

attgaagacg gtactcgttt ctcaatccgt gaaggtggac gtactgt 467

<210> 100
<211> 467
<212> DNA

<213> Staphylococcus warneri

<220>
<223> Strain N3

<400> 100
acaggccgtg ttgaacgtgg tcaaatcaaa gttggtgaag aagttgaaat cattggttta 60

catgacactt ctaaaacaac tgttactggt gtagaaatgt tccgtaagtt attagactac 120

gctgaagctg gtgacaacat cggtgcttta ttacgtggtg ttgctcgtga agacgtacaa 180

cgtggtcaag tattagctgc tcctggttca attacaccac atacaaaatt caaagcggaa 240

gtttacgttt tatctaaaga cgaaggtgga cgtcacactc cattcttcag taactaccgc 300

ccacaattct atttccgtac tactgacgta actggcgttg ttcaattacc agaaggtact 360

gaaatggtta tgcctggtga caacgttgaa atgacagtag aattaatcgc tcctatcgcg 420

attgaagacg gtactcgttt ctcaatccgt gaaggtggac gtactgt 467